# EUROPEAN PATENT SPECIFICATION

(11) **EP 3 253 353 B1**
(45) Date of publication and mention of the grant of the patent: **07.04.2021**
(21) Application number: 16747213.3
(22) Date of filing: 03.02.2016
(51) Int. Cl.: A61H 1/02, A61H 3/00

(54) **MEDICAL REHAB BODY WEIGHT SUPPORT SYSTEM WITH HORIZONTAL AND VERTICAL FORCE SENSING AND MOTION CONTROL**
KÖRPERGEWICHTTRAGESYSTEM ZUR MEDIZINISCHEN REHABILITATION MIT MESSUNG HORIZONTALER UND VERTIKALER KRÄFTE UND BEWEGUNGSSTEUERUNG
SYSTÈME MÉDICAL DE SUPPORT DE POIDS CORPOREL EN RÉÉDUCATION AVEC DÉTECTION DE FORCES HORIZONTALES ET VERTICALES ET COMMANDE DES MOUVEMENTS

(30) Priority: 03.02.2015 US 201562111288 P
(43) Date of publication of application: 13.12.2017
(73) Proprietor: Gorbel, Inc., Fishers NY 14453-0593 (US)
(72) Inventor: STOCKMASTER, James, G., Sodus, NY 14551 (US); PEETS, Brian, G., Fairport, NY 14450 (US); STROHMAN, Benjamin, A., Henrietta, NY 14467 (US); CHERNYAK, Alexnader, Z., Pittsford, NY 14534 (US); REESE, Blake, Honeoye Falls, NY 14472 (US); WRIGHT, Dean, C., Fairport, NY 14450 (US); LUO, Yi, Rochester Hills, MI 48307 (US); LIU, Li-Te, Taipei City 11047 (TW); DOLCE, Betty, Rochester, NY 14610 (US); FISCHER, Thomas, R., Wiliamsville, NY 14221 (US); BIERL, Chris, East Aurora, NY 14052 (US); POWERS, Judy, Orchard Park, NY 14127 (US); SACILOWSKI, Don, Lancester, NY 14086 (US)
(74) Representative: Williams Powell
(86) International application number: PCT/US2016/016414
(87) International publication number: WO 2016/126851

(56) References cited:
- US-A1- 2003 153 438
- US-A1- 2008 287 268
- US-A1- 2014 206 503
- US-B1- 6 315 138
- US-B1- 6 464 208

## Description

### TECHNICAL FIELD

The system disclosed herein relates to a body-weight support system, and more particularly to an improved support system, and method including exercise modes that are customizable or configurable and dynamic in nature, and which may include multiple configurations for a track system, where the system is capable of providing alternative functionality at differing locations, an adjustable and variable supportive force for users based upon, for example, a portion (e.g., percentage) of sensed body weight. The disclosed system further provides a user-interface that may be employed in a fixed, mobile, wired or wireless manner, and will enable the use of multiple support devices on a single track system. US2008287268 discloses a bodyweight support system and method. US2014/206503 discloses a medical rehab lift system and method with horizontal and vertical force sensing and motion control.

### BACKGROUND AND SUMMARY

The process of providing rehabilitative services and therapy to individuals with significant walking deficits and other physical impairments presents a challenge to even the most skilled therapists. For example, patients suffering from neurological injuries such as stroke, spinal cord injury, or traumatic brain injury often exhibit an inability to support themselves, poor endurance or walking patterns that are unstable. Such deficiencies make it difficult, at best, for the patient and therapist to engage in particular exercises, therapies, etc. Accordingly, it is increasingly common for such therapies to involve some sort of body-weight support system to reduce the likelihood of falls or other injuries, while enabling increased intensity or duration of the training or therapy.

Some existing support systems obstruct a therapist's interaction with the patient, by presenting barriers between the patient and the therapist. Other stand-alone support systems require assistance, or the patient, to manage the horizontal movement of the support system, rather than focusing on their own balance and preferred form of the therapy. In other words, the patient may be forced to compensate for the dynamics of the support system. Such a confounding effect could result in the patient's development of abnormal compensatory movements that persist when the patient is removed from the support system.

Yet a further problem with some systems is that under static unloading, the length of the supporting straps is set to a fixed length, so the subject either bears all of their weight when the straps are slack or no weight when the straps are taught. Static unloading systems are known to produce abnormal ground reaction forces and altered muscle activation pattern. Moreover, static unloading systems may limit the patient's vertical excursions (e.g., up and over steps, stairs and the like) and thereby prevent certain therapies where a large range of motion is required. Another problem observed with systems that are programmed to follow the patient's movement are significant delays in the response of the system (often the result of mechanics of sensors, actuators and system dynamics), where the patient feels that they are exerting greater force than necessary just to overcome the support system - resulting in the patient learning adaptive behaviors that may destabilize impaired patients when they ultimately begin self-supported activities for which they are being trained.

In light of the current body-weight support systems there is a need for a medical rehab support system and method that overcomes the limitations of the systems characterized above.

The invention pertains to a system for supporting a person as defined in claim 1. Preferred embodiments are set out in the appended dependent claims.

Disclosed in embodiments herein is a body-weight support system having an improved support system and method including exercise modes that are customizable or configurable and dynamic in nature, including numerous configurations for a track system, wherein the system is capable of providing alternative functionality at differing locations, an adjustable and variable supportive force for users based upon, for example, a percentage of sensed body weight. The disclosed system further provides a user-interface that may be employed in a fixed, mobile, wired or wireless manner, and the system will allow the use of multiple units on a single, possibly looped, track without collision or interference between adjacent units.

Further disclosed in embodiments herein is a system for supporting the weight of a person, comprising: a track or similar support structure including an indexed portion thereon (could also be supported by an arm or a gantry with ability to programmatically define a path over which the gantry trolley can move); a movable support operatively attached to the track, the support being movable along a path defined by the track and in a first direction and in a second direction generally opposite to the first direction; a first drive attached to the movable support, said first drive moving the support along the path defined by the track, wherein the first drive is operatively coupled to the indexed portion on the track to reliably control the horizontal position of the support along the track; an actuator attached to the movable support, said actuator including a second drive for driving a rotatable drum, said drum having a first end of a strap (or other flexible, braided member) attached thereto and the strap wound about an outer surface of the drum, with a second end of the strap being coupled to a support harness (or similar supportive/ assistive device) attached to support a person; a first sensor for detecting a horizontal force applied to the support via the strap; a second sensor for sensing a vertical force applied to the strap; and a control system configured to receive signals from the first and second sensors and a user interface and to control the movement of at least the first and second drives to facilitate the support and movement of the person, where the control system dynamically adjusts the amount of support provided to the person by altering at least the vertical force applied to the strap via the drum and second motor.

Also disclosed in embodiments herein is a system for supporting the weight of a person, comprising: a track including a plurality of extruded members joined end-to-end, and a plurality of electrical rails arranged longitudinally along an interior portion of the track for each portion of track, wherein at least one extruded member includes a generally planar upper surface extending in a longitudinal direction, opposing sides extending longitudinally and downward from each side of the upper surface, and where a combination of the upper surface and downward-extending sides form the interior portion of the track; each of said opposing sides further including a shoulder extending in an outward direction therefrom; a movable support unit operatively attached to the track, the movable support unit being movable along a path defined by the track in a first direction and in a second direction generally opposite to the first direction; a first drive attached to the movable support unit, said first drive moving the support along the path defined by the track, wherein the first drive is frictionally coupled to a surface of the track to control the horizontal position of the support along the track, wherein said first drive is maintained in frictional contact with the interior portion of the track and where the movable support unit is suspended from rollers resting on each of the shoulders extending from the opposing sides of the track; an actuator attached to the movable support unit, said actuator including a second drive for driving a rotatable drum, said drum having a first end of a strap attached thereto and the strap wound in an overlapping coil fashion about an outer surface of the drum, and a second end of the strap being coupled to a support harness attached to support a person; a first sensor for detecting a horizontal force applied to the movable support unit via the strap, including a strap guide operatively attached to and extending from said movable support unit, said strap guide being attached to a load cell in a manner causing a change in the load cell output when the strap is pulled in a direction forward from or backward from vertical; a second sensor for sensing a vertical force applied to the strap, including at least one pulley between the drum and the person supported by the strap, wherein the pulley is connected on one end of a pivoting arm, said arm being pivotally attached near its midsection to a frame member coupled to the movable support, and where an opposite end of said pivoting arm is operatively associated with a load cell such that the load cell is placed only in compression in response to a load suspended on the strap; and a control system configured to receive signals from the first and second sensors, and a user interface, and to control the movement of at least the first and second drives to facilitate the support during movement of the person, where the control system dynamically adjusts the amount of support provided to the person by moving the moveable support unit horizontally along the track to follow the person, thus minimizing the effect on the person, and by altering the vertical force applied to the person via the strap, the drum and second motor, to be suitable for a given patient.

Further disclosed in embodiments herein is A method for supporting the weight of a person for purposes of rehabilitation therapy, comprising: providing a track, the track including a plurality of extruded members joined end-to-end, and a plurality of electrical rails arranged longitudinally along an interior portion of the track for each portion of track, wherein at least one extruded member includes a generally planar upper surface extending in a longitudinal direction, opposing sides extending longitudinally and downward from each side of the upper surface, and where a combination the upper surface and downward-extending sides form the interior portion of the track; each of said opposing sides further including a shoulder extending in an outward direction therefrom; operatively attaching a movable support unit to the track, the movable support unit being movable along a path defined by the track in a first direction and in a second direction generally opposite to the first direction; moving the support unit along the path defined by the track using a first drive attached to the movable support unit, wherein the first drive is operatively coupled to a surface of the track to control the horizontal position of the support along the track, and where the movable support unit is suspended from rollers resting on each of the shoulders extending from the opposing sides of the track; controlling the vertical position of the person using an actuator attached to the movable support unit, said actuator including a second drive for driving a rotatable drum, said drum having a first end of a strap attached thereto and the strap wound in an overlapping coil fashion about an outer surface of the drum, and a second end of the strap being coupled to a support harness attached to support the person; detecting a horizontal force applied to the movable support unit via the strap using a first sensor, the first sensor including a strap guide operatively attached to and extending from the movable support unit, the strap guide being attached to a load cell in a manner causing a change in the load cell output when the strap is pulled in a direction forward from or backward from vertical; sensing a vertical force applied to the strap using a second sensor, the second sensor including at least one pulley between the drum and the person supported by the strap, wherein the pulley is connected on one end of a pivoting arm, said arm being pivotally attached near its midsection to a frame member coupled to the movable support, and where an opposite end of said pivoting arm is operatively associated with a load cell such that the load cell is placed only in compression in response to a load suspended on the strap; and providing a control system configured to receive signals from the first and second sensors, and a user interface, and to control the movement of at least the first and second drives to facilitate and support movement of the person, where the control system dynamically adjusts the amount of support provided to the person by moving the moveable support unit horizontally along the track to follow the person.

### BRIEF DESCRIPTION OF THE DRAWINGS

FIG. 1 is a representation of an exemplary rehab support system;
FIG. 2 is an illustration of a support harness assembly with a person in the harness;
FIG. 3 is a view of a support on a section of track in accordance with a disclosed embodiment;
FIG. 4 is a side view of a support on a section of track in accordance with an alternative embodiment;
FIG. 5 is a cutaway end view along section 5-5 of FIG. 4;
FIG. 6 is a cutaway end view along section 6-6 of FIG. 4;
FIG. 7 is a perspective view of one of the support suspension assemblies of the embodiment of FIG. 4;
FIGS. 8 and 9 are, respectively, perspective and top views of the frictional horizontal drive of the embodiment of FIG. 4;
FIG. 10 is a perspective view of the embodiment of FIG. 4, including components on the interior of the track;
FIG. 11 is an enlarged view of a portion of the support including components of the vertical body weight support system;
FIG. 12 is a perspective view of the drum used to wind the strap in the system of FIG. 11;
FIG. 13 is a perspective view of a strap slack/tension sensing system;
FIG. 14 is perspective view of a vertical drive, drum and strap sensing system in accordance with the support embodiment of FIG. 4;
FIG. 15 is an enlarged view of the strap slack and tension sensing system in accordance with the embodiment of FIG. 4;
FIG. 16 is an illustration of the control flow for a disclosed embodiment of the rehab support system;
FIGS. 17 and 18 are exemplary illustrations of a generally rectangular track system;
FIGS. 19 - 21 are illustrative examples of user interface screens for controlling basic operations of the rehab body weight support system;
FIGS. 22 - 23 are illustrative examples of user interface windows for tracking and entering patient-specific information relating to use of a rehab body weight support system;
FIG. 24 is an illustrative example of a user interface day list window;
FIG. 25 is an illustrative example of a user interface plan of care window;
FIG. 26 is an illustrative example of a user interface window for review and entry of data for a patient session;
FIGS. 27 - 31 are illustrations of a software environment for embodiments including a kiosk and remote applications for system control and data storage; and
FIGS. 32 - 56 are illustrative screenshots for several features of the system that are available via embodiments including an interface such as the kiosk and/or remote application interfaces.

The various embodiments described herein are not intended to limit the disclosure to those embodiments described. For a general understanding, reference is made to the drawings. In the drawings, like references have been used throughout to designate identical or similar elements. It is also noted that the drawings may not have been drawn to scale and that certain regions may have been purposely drawn disproportionately so that the features and aspects could be properly depicted.

### BEST MODE FOR CARRYING OUT THE INVENTION

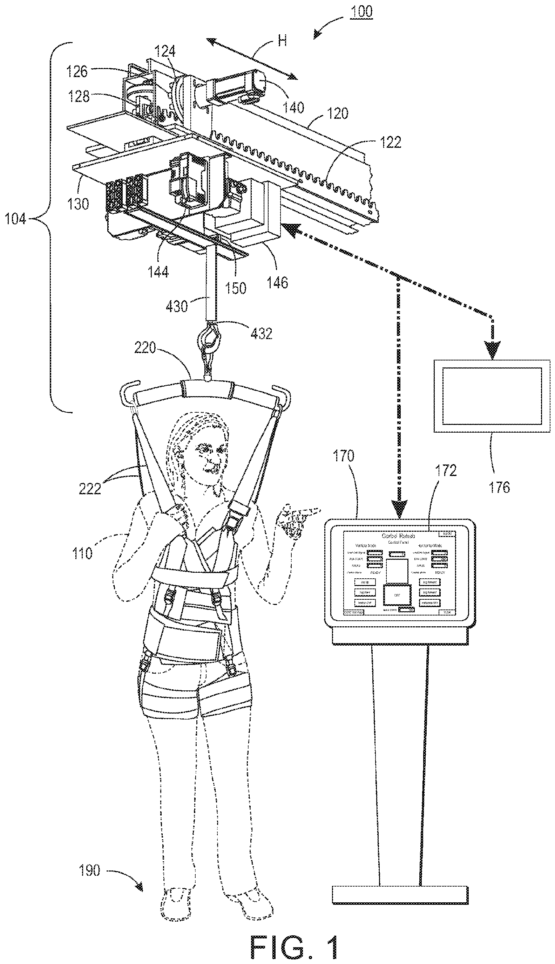

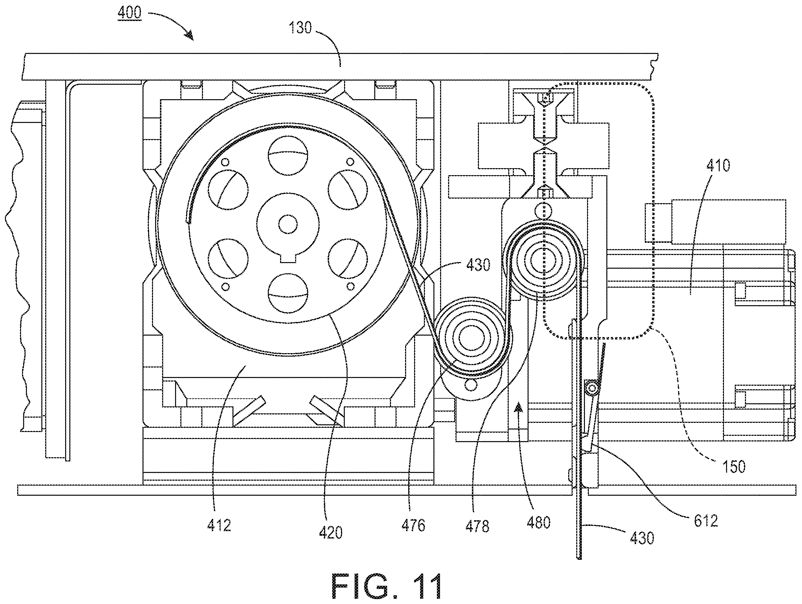

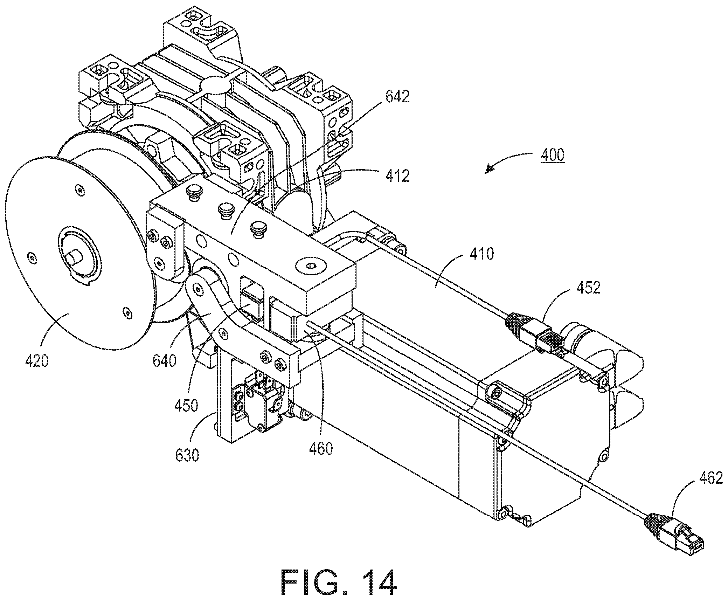

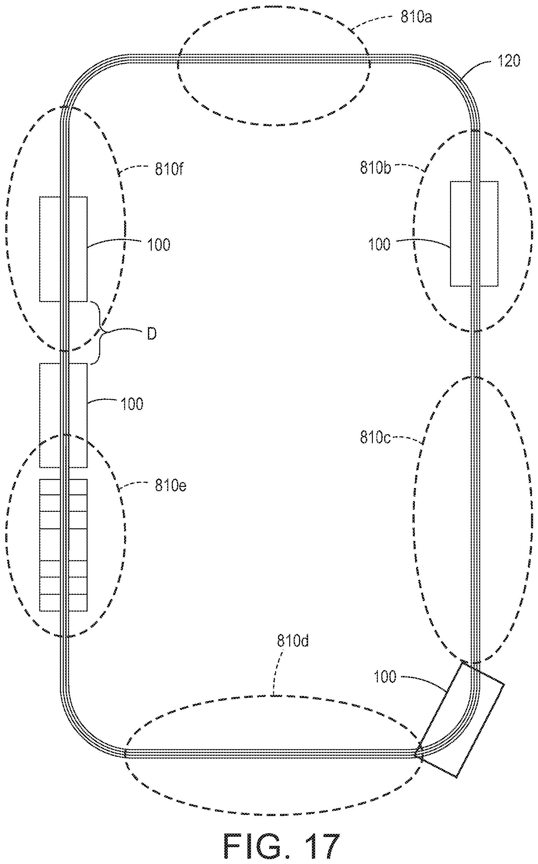

Referring to FIG. 1, depicted therein is a system **100** (e.g., SafeGait™ 360° Balance and Mobility Trainer system) for supporting a selectable portion (e.g., percentage) of the weight of a person or patient 110. In a general sense, the system comprises a track **120.** Although the following disclosure is largely directed to a track-type system, for example a looped track path as illustrated in FIGS. 17 - 18 (e.g., no-beginning or end), various aspects and features of the disclosed system and associated methods are contemplated as being supported by alternative support structures such as an arm (e.g., Jib crane, Gorbel EasyArm™), a cantilevered track section, and perhaps even a gantry with the ability to programmatically define a path over which the gantry trolley can move. In such alternative embodiments, a movable support unit or truck **104** includes a movable support or base **130,** where the support **130** may be fixed to another movable member or may itself be movable relative to a supporting structure. The movable support unit further includes other components such as a horizontal drive **140,** actuator **400,** etc. as will be further described below (e.g., FIGS. 11, 14).

The movable support **130** is, in the embodiment of FIG. 1, operatively attached to the track **120,** the support being movable along a path defined by the track. Moreover, the generally horizontal movement (H) of the support relative to the track or path along a longitudinal or central axis of the track or track section, and may be in both a first direction and in a second direction generally opposite to the first direction. While illustrated as a horizontal track over which the support **130** travels, also contemplated is a track system where one or more portions or sections of track **120** may be raised or lowered relative to the remainder of the track and/or where a surface or flooring **190** beneath the track is raised or lowered at varying positions, so as to provide or simulate typical scenarios where the person is proceeding up or down an incline, stairs, curbs, etc.

Continuing with FIG. 1, a first or horizontal drive **140** is attached to the movable support, and the first drive includes, in one embodiment, a pinion **124** configured to interact with the toothed indexing portion or rack and in response to the rotational motion of the drive **140,** the support is moved along the path defined by the track. As will be appreciated, the horizontal drive is thereby operatively coupled to the indexed portion on the track to reliably control the horizontal position of the support along the track. Using an appropriate drive, for example a servo drive motor provided by B&R (Model #8LS35), it is possible to be relatively precise in both controlling and monitoring the position of the drive and support. More specifically, due to the relationship of the pins or lugs **126** on the pinion **124,** and the direct coupling of such pins to the "teeth" on the rack **122,** any angular rotation of the pinion under the control of the motor will advance or retract the position of the support along the track.

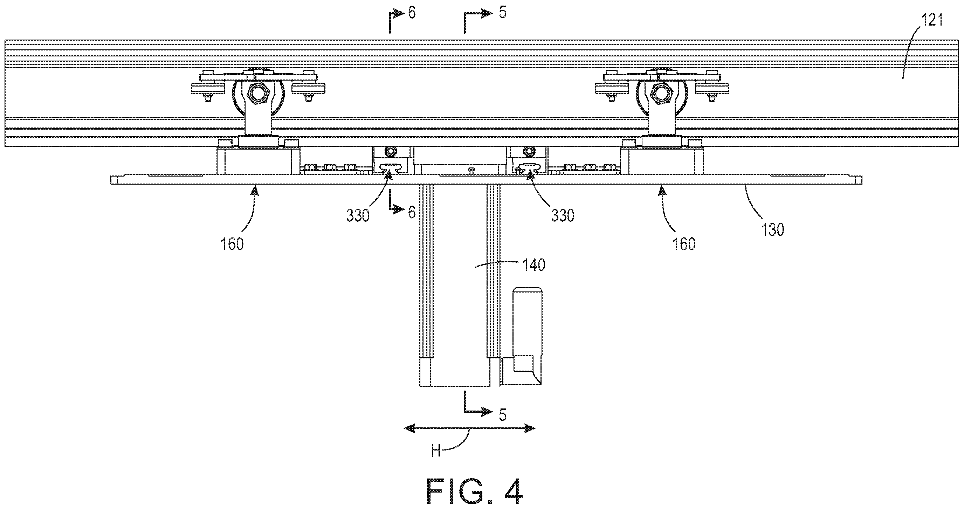

In contrast, in the alternative embodiment depicted in FIGS. 4 - 10, the horizontal drive **140** may be frictionally engaged with a surface (e.g., interior wall) of track **120.** By driving along an interior wall, the system reduces the likelihood of debris interfering with the frictional drive. As will be appreciated, the operation of the horizontal drive **140** is controlled by a AC servo drive **144,** or similar device that is both under programmatic control and further receives signals controlling its operation, for example via a horizontal force sensing assembly **150** and/or via a programmable device such as an industrial PC **170** including a user interface **172** such as that depicted by reference numeral **170** in FIG. 1. Power is supplied to the servo drive **144** via power supply **146.**

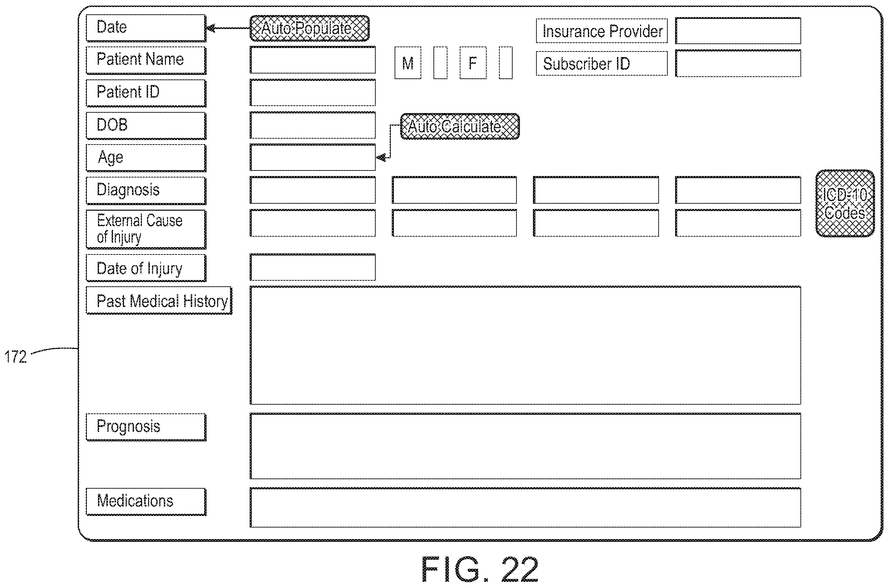

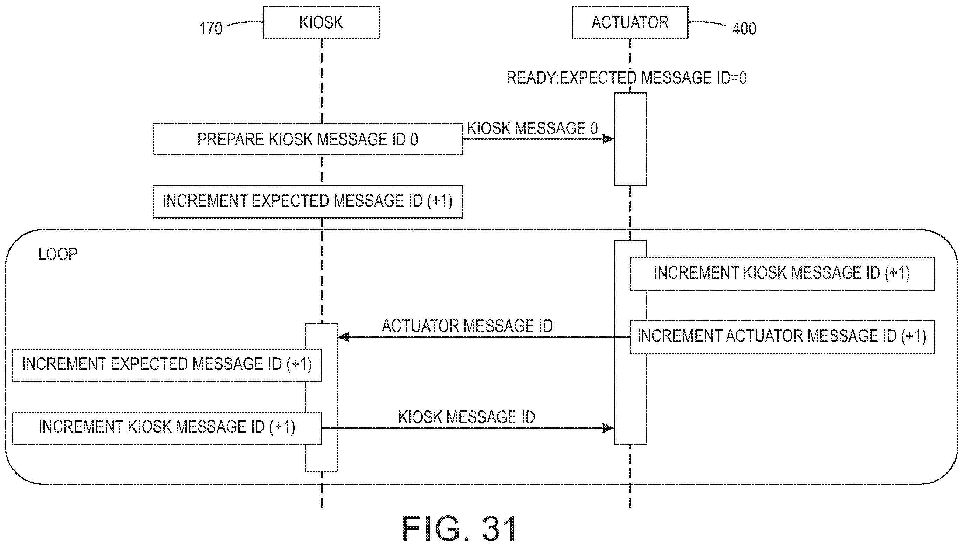

Although depicted as a floor-mounted device, industrial PC **170** may take one or more forms and may be portable, floor-mounted, and may also include remote-control devices such as tablet **176.** For example, controller **170** may be a programmable logic controller, available from B&R (Model #PP500). In one embodiment, while there may be a main or centralized control point, that control point may consist of or include a wireless transceiver to communicate with one or more handheld devices (smart phones, tablets, or customizable controllers) that are able to remotely control the operation of the system. Controller **170** may further include memory or storage devices suitable for recording information relating to system usage, patient information, etc. Wireless communications techniques may employ one or more radio frequencies (e.g., Bluetooth), as well as other bandwidth spectrums such as infrared. In one embodiment, the disclosed system may employ an Ethernet or similar communication protocol and technology to implement communications between the various system components. In this manner, a therapist or person attending the patient **110** may be able to control the operation of the device, select, set or modify a program for the patient, etc. as further represented in FIGS. 22 - 56. In other words, the therapist may be able to modify or change parameters associated with a patient on the fly using a kiosk, handheld tablet, etc., where using the touch-screen display of interface **172** or similar wireless remote interface **176** the operation of the movable support **104** may be controlled or adjusted. It is also contemplated that the communications may be of a wired nature between a controller **170** and AC servo drive **144.**

The following is an exemplary representation and description of an exemplary software design of the SafeGait™ 360° Balance and Mobility Trainer system **100.**

### 1 Introduction

### 1.1 Scope

This description provides design detail for the custom software modules of the SafeGait 360° Balance and Mobility Trainer system (see "System Overview," below). Specifically disclosed are designs of the interfaces, structures, and implementation specifics of the Kiosk (e.g., **170**) and Remote (e.g.,**176**) user interfaces (e.g. **2900**) and the supporting software components upon which they depend for integrating and functioning within the system as a whole.

### 1.2 Objectives

This document is intended to provide the implementation details of the software components of the system, the reasoning for their design structure, and how these modules integrate with the SafeGait hardware and firmware.

### 2 Design

### 2.1 Considerations and Constraints

Several constraints exist for this system, as defined within the system requirements (SRS), which drove the design: (1) The user interfaces support a touchscreen; (2) The Kiosk and Remote hardware may not run the same operating system platform; (3) Aside from providing a TCP communications interface, the Actuator is considered a *black box*; (4) The system may accommodate future "smart" technologies, (e.g., Google Glass, Smart Watch, etc.); (5) The system should be capable of supporting future Kiosk and Remote hardware; and (6) The system should support future use cases, such as data concurrency between multiple systems at a single facility. Together, these considerations lead to the architecture of a cross-platform, web-based approach based on loose coupling between software and hardware components, a top-level *model-view-controller* (MVC) component structure, and the use of industry-standard technologies to ease integration and expandability.

### 2.2 Top-Level Approach

The requirements noted above may require the user interfaces to change if operating platforms change or are added, the data modeling strategy to change if a centralized data store becomes necessary, or the interface to the *black box* Actuator to change. As such, being able to decouple one software component from another based on their roles within the system becomes a critical requirement - the user interface *should not* embed communications code or data management code within its source. Instead, the user interface *should* invoke another component, using an agreed-upon contract, to execute a message to the Actuator, or fetch a record from the data store. Thus, the design approach takes advantage of the fact that the software system can effectively be segregated into the following MVC categories: Kiosk and Remote User Interfaces ("view"); Patient and User Data Store ("model"); and Communications and Background/Supporting Tasks ("controller")

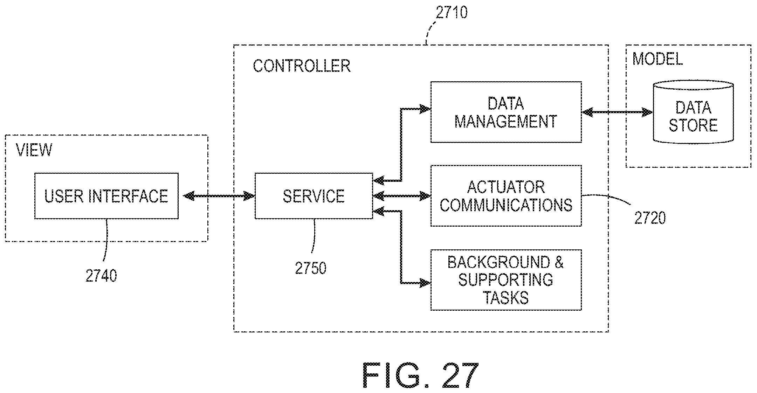

The top-level *separation of concerns* (SoC) outlined above allow any piece of the various software components comprising the system to be updated, changed, or even re-written with minimal impact on the rest of the components. This organization is summarized in FIG. 27, with the component labeled "Service" managing the requests and responses from the other components of the system (this component will be discussed in detail later in the document).

As characterized by FIG. 27, which depicts the MCV software architecture, within each *model-view-controller* category, the same SoC paradigm is also maintained. For instance, within the *controller* component **2710,** the communications module **2720** that interfaces with the Actuator will be maintained as a different entity (e.g., library) from the module that communicates with the user interfaces **2740.** This allows for the Actuator's protocols to change without needing to change the entire controller component. This also presents the *software as a service* design pattern, wherein a specific module or entity is only activated when its tasks are required. This type of segregation also lends itself well to the use of web-based technologies, which is also a favorable approach to multiple aspects of the elucidated set of system requirements:
1. When considering that the operating systems utilized by the Kiosk and Remote hardware components may differ, a web-based user interface better lends itself toward a consistent look and feel and functional behavior across platforms;
2. Web technologies are designed to communicate between different systems - many different data formatting protocols as well as transport protocols are built into the web platforms;
3. Web technologies provide comprehensive support for many types of data storage platforms; and
4. Web technologies guarantee scalability to more systems, more components, and more data.

Thus, the controller component is implemented as a web service (e.g., the "Service" module **2750** in FIG. 27), to which the web application comprising the user interface sends requests and receives responses, and which invokes the functions necessary for data and system management. The details of the implemented structure of these components are elucidated in the "System Overview" section below.

### 2.3 Privacy and Security Risks

As a training device, patient privacy and patient data security is always of concern. This category of risks may be mitigated both by user training as well as within the software and its configuration, including the following: Persistent data stores will segregate identifiable fields (e.g. name, date of birth, etc.) into their own tables, and pursue the identification of a patient throughout the system using unique, secondary identifiers; Persistent data stores will encrypt fields that may be considered identifiable; The user interfaces will require authentication and authorization to access system data; The system will run on a closed, secured, private network; and The user interface will not display sensitive, identifiable information when not necessary.

### 2.4 Development Platforms

In one exemplary embodiment, the Kiosk platform is based upon a Windows 8.1 Professional, 64-bit computer. This selection drives the technology baseline of the architecture. For example, for the purpose of better integrating with Microsoft technologies, the following configurations and their corresponding software components may be used:
Microsoft .NET 4.5 Framework with C#
Frameworks:
   - Entity Framework 6 - Used for data modeling and management
   - WebAPI Framework - Used for Web Service implementation
Infrastructure:
   - Internet Information Services (IIS) 7.0 - Used to host web service
   - SQL Server 2014 Express - Used as data store
   - Chrome Browser 39 or later

Because the user interfaces are essentially web applications that are compiled to different target platforms, Microsoft technologies may not be suitable. Instead, a more platform-agnostic development configuration is considered such that the cross-compilation toolchain, Cordova, may be used to create native application packages for the target environments, where applicable. Additionally, the use of a responsive design template (e.g., Bootstrap) allows for automatic reconfigurations of the user interface depending on the detected screen size, such as between the Kiosk **170** and the Remote **176.**

The combination of these technologies - generic web application, responsive styling template, and a cross-compiling toolchain - enables code reuse between the Kiosk and Remote components and easier source code maintenance. The following underlying frameworks are used for the user interfaces:
Web Application:
∘ HTML5 - Used for screen layouts
∘ CSS3 (with Bootstrap 3 template) - Used for user interface look and feel
∘ JavaScript - Used as the backend infrastructure as well as for client-side behavior jQuery - Core library of common function (dependency of many libraries used) AngularJS application infrastructure with the following plugins:
   - ngResource (angular-resource) - For interacting with RESTful services
   - ngCookies (angular-cookies) - For reading/writing browser cookies
   - ngSanitize (angular-sanitize) - For operating with well-formed HTML
   - ngAnimate (angular-animate) - For support for CSS3 animations
   - ngTouch (angular-touch) - For touch event support (i.e., touchscreens)
   - uiRouter (angular-ui-router) - For managing navigation hierarchies
   - uiMask (angular-ui-utils) - For input validation
   - angular-base64 - For supporting Base64 encoding
   Date/Time tools:
   - MomentJS - Parsing, validating, and displaying dates
   Charting tools:
   - C3 charts (using D3 technology)
   Other utilities:
   - MathJS - Used for formatting and calculations not provided by default Cordova for cross-compiling to Kiosk and Remote operating platforms

### 3 Environmental Configurations

### 3.1 Kiosk Hardware

The Kiosk and/or the Remote hardware may be a Windows 8.1 Professional tablet that supports a touchscreen. It needs to have IIS installed and be configured with the capability of running the web service component of the system such that external entities may connect to it.

Specific Kiosk configuration details include tasks such as operating system restrictions, installation of software, and configuration of users, rights, and privileges. From a software systems perspective, the kiosk's wireless network adapter will be set up to automatically connect to the "SafeGait" network broadcasted by the wireless router (mentioned below.) The kiosk will be configured to use a defined IP address and subnet mask.

### 3.2 Remote Hardware

The Remote hardware should be small enough such that a user can comfortably hold it in one hand, thereby leaving the other hand free to assist the patient. Additionally, the software platform running on the Remote hardware should have the following capabilities:
- Connecting to the "SafeGait" wireless (WiFi) network
- Setting a static Internet Protocol (IP) address
- Touchscreen support
- Supported by Cordova

### 3.3 Wireless Router

A standard wireless router will be used to create the private network between the actuator, kiosk, and remote. The router will be configured with a known SSID and WPA key. The router's IP address and subnet will be predefined.

### 3.4 Network Attached Storage (NAS)

The Network Attached Storage is designed to be used as a data storage and backup unit and will employ configuration restrictions similar to the Kiosk and Remote operating platforms.

### 4 Software System Design

### 4.1 System Overview

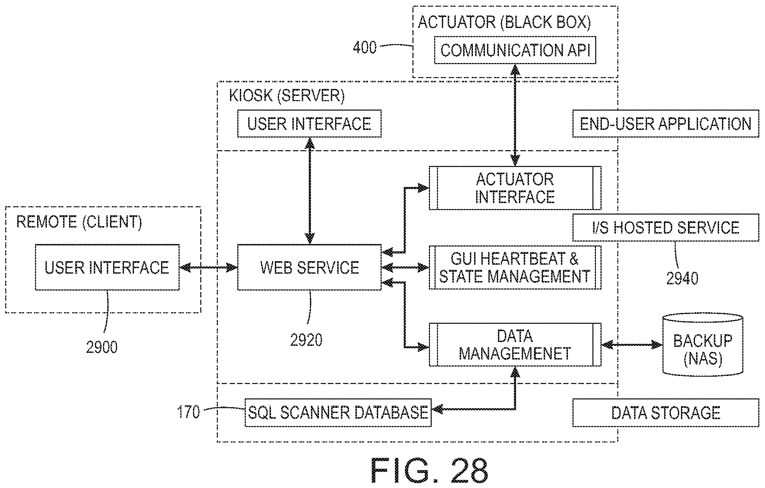

Based on the system requirements, the SafeGait 360° software system was designed with the components of FIG. 28. FIG. 28 illustrates major components by their task responsibilities and where they reside in the hierarchy of modules. It is important to note that within the "IIS Hosted Service" space 2940, the items labeled "Actuator Interface," "GUI Heartbeat & State Management," and "Data Management" may be physically separate (e.g., the Actuator Interface DLL), or structurally part of the web service (e.g., GUI State Management). The key is that the Web Service component **2920** is responsible for exposing and managing multiple backend tasks relegated to the server (Kiosk) within the system.

Not pictured in the diagram is a logging module, which may be a separate entity that is invoked by multiple subcomponents of the system. The logging module writes files to the database in a looped structure, such that the most recent information regarding actions undertaken within the system may be reviewed. Further details on each of the components depicted in FIG. 28 may be found in the sections below.

### 4.1.1 Safety Considerations

As a training device whose use requires that a live patient be attached to the system, safety is of utmost concern. The software components are not directly responsible for how the harnessed individual is managed while the system is in use insofar as it is the Actuator's firmware that has direct control. However, the system serves to relay requests from the therapist to the Actuator, thereby indirectly impacting safety. Thus, the following safety considerations, as defined within the system requirements, are reiterated here:
1. The system must always be aware of whether the Actuator is present;
2. There must be a way to forcibly and immediately tell the Actuator to stop movement;
3. There must be a way to know which user interface (Kiosk versus Remote) has control of the Actuator;
4. Should auxiliary control interfaces (e.g., the Remote) lose connection, a primary control interface (e.g., the Kiosk) needs to automatically regain control;
5. The user interface must adequately be able to display any perceived issues from any system component to the end user; and
6. As an audit trail, actions taken by the software system should be logged for offline evaluation.

### 4.2 System Interfaces

As seen in FIG. 27, the various software components of the system require the capability to communicate with each other to relay information, to command the Actuator, and to persist system data. Specifically, the user interfaces on the Kiosk and Remote utilize the Web Service to interface with the rest of the system. The Web Service, in turn, invokes various different modules in order to relay messages to and from the Actuator, send and retrieve data from the data store (e.g., database), and to manage various other low-level system states and tasks.

To affect the communications, the system is designed to communicate over the TCP/IP protocol, within a closed, wireless network. In one embodiment the Web Service uses JSON-formatted text as the scheme for message transmission over HTTP. Details on the Web Service's design, responsibilities, and functions are discussed below. It will, however, be appreciated that alternate schemes may be employed for message transmission and communications between or within the system components.

Communications with the Actuator (**2720**) employs the creation of connectionless, UDP-style datagram packets as defined by the provided (black box) Actuator API before being pushed over a TCP transport layer. Communications with the backend database is accomplished using Microsoft's built-in data frameworks. However, the underlying technology behind the transmission of data functions also utilizes TCP. This layer is not discussed in detail as it is encapsulated through Microsoft's programming libraries. The structure and design of the data layer itself, however, is discussed below.

### 4.3 Background Tasks

### 4.3.1 Admin Service

This windows service is responsible for handling the exit and shutdown requests from the Kiosk user interface. When the user interface receives a request to exit or shutdown, it is forwarded to the Web API, which sends the command signal to the windows service. The windows service then performs the necessary action under the appropriate privileges. This service is automatically started when the operating system boots up.

### 4.3.2 Database Backup Scheduled Task

The data store for patient and session information is routinely backed up to the attached NAS unit. This is a system-level configuration to run the backup procedure as a Windows-level Scheduled Task. Thus, the operating system becomes responsible for the execution of backup, and logs successes and failures automatically.

### 4.4 WebAPI

The WebAPI is the entity responsible as the communications pipe between the different components of the software, as well as between the software and the Actuator. This is a managed set of libraries that are divided into functional groups, as described below. In one embodiment live documentation of each API function, as well as its usage, can be found on the server on the Kiosk by navigating to an appropriate URL.

### 4.4.1 User Management

.NET SimpleMembership may be used to support or administer the user accounts of the system. Three roles will be created to restrict access to certain features of the system: Therapist (user); Admin (admin); and Service Technician (superuser).

### 4.4.1.1 User Account API Resources

### 4.4.1.1.1 Login

Log user into system. This user is given system control.

### 4.4.1.1.2 Logout

Log user out of system. Since there is no longer system control, the system is disconnected from the actuator.

### 4.4.1.1.3 Add User

Add a new user account to the system. Admin or Service Technician role required.

### 4.4.1.1.4 Update User

Update existing user account details. Admin or Service Technician role required.

### 4.4.1.1.5 Reset Password

Reset user account password to known default password; Admin or Service Technician role required.

### 4.4.1.1.6 Change Password

Allow any user to change their password.

### 4.4.1.1.7 Disable/Enable User

Disable/Enable user accounts. This has the effect of preventing users from logging into the system. Admin or Service Technician role required.

### 4.4.2 Data Management

### 4.4.2.1 Patient API Resources

The patient resources are provided to allow the management or administration of patient information as may be stored in the database.

### 4.4.2.1.1 Add Patient

Add a new patient record.

### 4.4.2.1.2 Update Patient

Update an existing patient record.

### 4.4.2.2 Session API Resources

### 4.4.2.2.1 Add Session

Associate a new session, or "Plan of Care," with an existing patient record.

### 4.4.2.2.2 Update Session

Update an existing patient session.

### 4.4.2.3 Predefined and Custom Tasks API Resources

### 4.4.2.3.1 Get Tasks

Retrieve a listing of predefined tasks that can be performed by the patient.

### 4.4.2.3.2 Add Custom Task

Add a new custom task to the set of available tasks.

### 4.4.2.4 Historical Data API Resources

### 4.4.2.4.1 Get Patient Sessions

Retrieve a listing of the completed patient sessions, and their performed tasks.

### 4.4.2.4.2 Get Patient Goals (by task type)

Retrieve a historical listing of the patient's goals by task type.

### 4.4.2.4.3 Get Session Task Metrics (by task type)

Retrieve a historical listing of task metrics by a given task type.

### 4.4.3 System Management

### 4.4.3.1 Background Tasks

### 4.4.3.1.1 Heartbeat Monitor

The heartbeat monitor task tracks the last heartbeats from the kiosk and remote user interfaces. If either the kiosk or the remote has control of the system, and the monitor does not receive a heartbeat in a required interval, system control is released from that device. The monitor logs any of these notable events to the database.

### 4.4.3.1.2 Actuator Connectivity Monitor

The actuator activity monitor is responsible for tracking two system events. In order for the system to be connected to the actuator, a user needs to be logged in at the kiosk. If the system loses actuator connectivity while a user is logged in, this task attempts to re-establish connectivity with the actuator. If a user logs out of the kiosk, this task also ensures that the system disconnects from the actuator.

### 4.4.3.2 System API Resources

### 4.4.3.2.1 Kiosk/Remote Heartbeat

The kiosk and remote user interfaces will invoke this API resource to report into the system. The response to this request is a snapshot of the system's state: who is logged in, what device has control, and the UI session data of the controlling source. This is how synchronization is facilitated between the kiosk and remote.

### 4.4.3.2.2 Grab System Control

Only one device, kiosk or remote, may have control of the system at a time. Either device can take control from the other. This API resource is used to take control of the system.

### 4.4.3.2.3 Throw System Control

Throwing system control is typically performed when a device's heartbeat is lost. When this happens, this API resource is used to notify the other user interface that it can automatically regain control of the system.

### 4.4.3.2.4 Get/Set Application Settings

Retrieve and update application settings.

### 4.4.3.2.5 Exit App/Shutdown

Sends the exit or shutdown signal to the Admin Service.

### 4.5 Actuator Interface

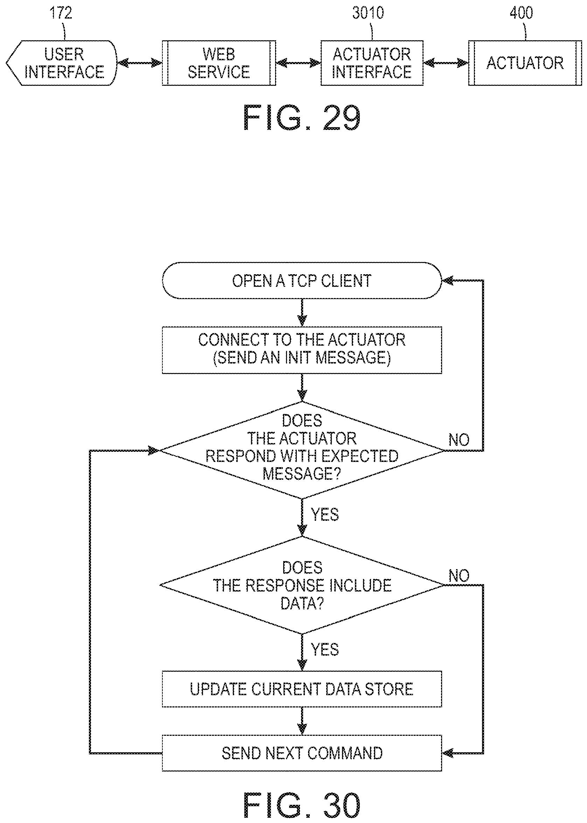

The Actuator Interface (AI) **3010** serves as the integration point between the Web Service and the Actuator. This integration is effectively the processing center where requests and their responses between the User Interfaces and the Actuator are encoded and decoded as they are relayed through the Web Service. Figure 29 depicts the Actuator Communications Relay

### 4.5.1 Development Configuration

The Al may be written in C# using the Microsoft .NET 4.5 Framework. It is compiled as a library (DLL) and referenced by the Web Service.

### 4.5.2 Command Packets

Requests and responses are sent in the form of commands, comprising a header section followed by an optional data component. Together, the two components form a datagram package. For the packet formats the following applies:
- Header Format:
- Data Format:
   ∘ The data section may include information sent to, as well as received from, the Actuator
   ∘ The data section includes both read and write areas
   ∘ The data section comprises a fixed length, which is always sent in its entirety
   ∘ A command packet does not always contain a data section

### 4.5.3 Control Loop

Upon initial communication with the Actuator **400,** this interface establishes and maintains consistent communications with the Actuator, as depicted in detail in FIGS. 30-31. Note that the detail of the handling of special case commands, such as the Shutdown command, is not depicted in the figure.

### 4.5.3.1 System Heartbeat

As mentioned above (see section "Safety Considerations"), a system heartbeat is required for the various user-interfacing software components to remain active. The Al is responsible for initiating and maintaining the system heartbeat after initial communications with the actuator have been established.

To affect this heartbeat, the last good command (without data, if applicable) must be resent to the actuator at intervals of at most two (2) seconds.

### 4.5.4 Actuator Interface Functions

The Actuator is capable of many actions, of which the Kiosk and Remote user interfaces will only utilize a small subset. Actuator commands and capabilities include the following subset of commands used within this system:

### 4.5.4.1 Interrogate

Test network connectivity with actuator.

### 4.5.4.2 Connect

Create network connection with actuator and initiate heartbeat.

### 4.5.4.3 Disconnect

Stop heartbeat with actuator and terminate network connection.

### 4.5.4.4 Reset

Reestablish network connection and heartbeat with actuator.

### 4.5.4.5 StopAll

Send "stop all" command to actuator.

### 4.5.4.6 Move

Send directional move command (velocity mode) to actuator.

### 4.5.4.7 Stop

Send directional stop command (velocity mode) to actuator.

### 4.5.4.8 GetStatus

Retrieve overall status information: connectivity, settings, mode, and task status information.

### 4.5.4.9 GetVersion

Retrieve the actuator version information.

### 4.5.4.10 GetSerialNumber

Retrieve the actuator serial number.

### 4.5.4.11 BeginTask

Send the start task command (float mode) to actuator.

### 4.5.4.12 EndTask

Send the stop task command (float mode) to actuator.

### 4.5.4.13 EnableFloatMode

Toggle float mode enabled state.

### 4.5.4.14 EnableDescentLimit

Set descent limit enabled state in float configuration.

### 4.5.4.15 SetRepetitionLowerLimit

Send repetition lower limit "set" command to actuator.

### 4.5.4.16 SetRepetitionUpperLimit

Send repetition upper limit "set" command to actuator.

### 4.5.4.17 SetDescentLimitHeight

Set descent limit height in float configuration.

### 4.5.4.18 SetBodyWeightSupport

Set body weight support value in float configuration.

### 4.5.4.19 SetDfpSensitivity

Set DFP sensitivity level (high, medium, low) in float configuration.

### 4.5.4.20 ApplyBoost

Send boost command to actuator.

### 4.5.4.21 ClearError (or warning)

Clear the last error or warning flags.

### 4.5.4.22 ClearFall

Clear the last fall flag.

### 4.6 Database

Primary keys will be denoted with PK. Foreign keys will be denoted with FK. If a primary key cluster exists, multiple fields will be denoted with PK. All fields are required unless specified as "optional."

### 4.6.1 Configuration

| **ApplicationParameter** | | |
|---|---|---|
| Field | Datatype | Notes |
| Parameterld | string | PK |
| Description | string | |
| DefaultValue | string | |

| **Site** | | |
|---|---|---|
| Field | Datatype | Notes |
| Siteld | integer | PK (identity) |
| ShortName | string | unique |
| DisplayName | string | unique |

| **SiteParameter** | | |
|---|---|---|
| Field | Datatype | Notes |
| Siteld | integer | PK, FK |
| Parameterld | string | PK, FK |
| Value | string | |

### 4.6.2 User Management

| **UserProfile** | | |
|---|---|---|
| Field | Datatype | Notes |
| Userld | integer | PK (identity) |
| UserName | string | unique |
| Email | string | unique |
| FirstName | string | |
| LastName | string | |
| Title | string | optional |
| Disabled | boolean | |

### 4.6.2.1 .NET SimpleMembership Supporting Tables

SimpleMembership utilizes the default, auto-generated tables to complement the User Profile table. These tables are not described in detail, as they are the standard schemas provided by the .NET Framework: webpages_Membership; webpages_Roles; webpages_UserInRoles; andwebpages_OAuthMembership.

### 4.6.3 Patient Management

The following table tracks patient profiles.

| **Patient** | | |
|---|---|---|
| Field | Datatype | Notes |
| Patientld | integer | PK (identity) |
| FirstName | string | |
| LastName | string | |
| DateOfBirth | date | |
| Gender | string | |
| Height | real | inches |
| Weight | real | pounds |

The following tables will support all the optional patient fields in the user interface.

| **PatientAttri bute** | | |
|---|---|---|
| Field | Datatype | Notes |
| Attributeld | string | PK |
| Description | string | optional |

| **PatientAttribute Value** | | |
|---|---|---|
| Field | Datatype | Notes |
| PatientId | integer | PK, FK |
| Attributeld | string | PK, FK |
| Value | string | I |

### 4.6.4 Session Management

| **Session** | | |
|---|---|---|
| Field | Datatype | Notes |
| Sessionld | integer | PK (identity) |
| Patientld | integer | FK |
| Userld | integer | FK |
| StartTime | datetime | optional |
| EndTime | datetime | optional |

| **SessionTask** | | |
|---|---|---|
| Field | Datatype | Notes |
| SessionTaskld | integer | PK (identity) |
| SessionId | integer | FK |
| TaskId | integer | FK |
| UserId | integer | FK |
| StartTime | datetime | optional |
| EndTime | datetime | optional |
| TimeGoal | real | optional |
| DistanceGoal | real | optional |
| RepetitionsGoal | int | optional |
| Notes | string | optional |

### 4.6.5 Task Management

| **Task** | | |
|---|---|---|
| Field | Datatype | Notes |
| TaskId | integer | PK |
| Name | string | unique |
| Category | string | |
| Description | string | optional |
| HasDistance | boolean | |
| HasRepetitions | boolean | |

| **TaskMetric** | | |
|---|---|---|
| Field | Datatype | Notes |
| MetricId | string | PK |
| Label | string | |
| Description | string | optional |

| **TaskMetricValue** | | |
|---|---|---|
| Field | Datatype | Notes |
| SessionTaskld | integer | PK, FK |
| Metricld | string | PK, FK |
| Value | real | |

### 4.6.6 Logging

| **Log (log4net)** | | |
|---|---|---|
| Field | Datatype | Notes |
| Id | integer | PK |
| Timestamp | string | |
| Thread | string | |
| Level | string | |
| Logger | string | |
| Message | string | |
| Exception | string | |

### 4.7 User Interface

### 4.7.1 Kiosk

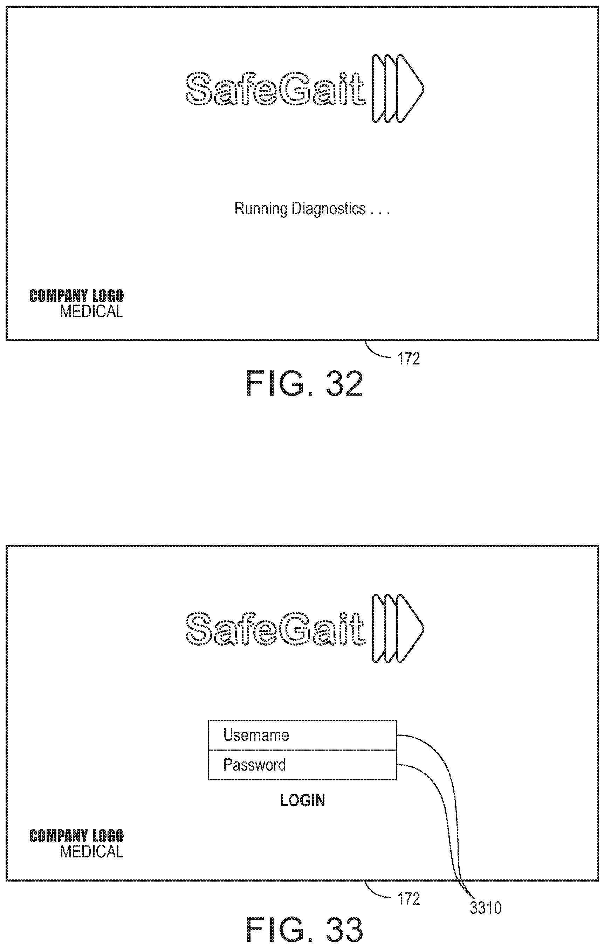

The Kiosk user interface application is an AngularJS web application hosted locally on the kiosk laptop. AngularJS is a Javascript MVC framework used for building client-side web applications (apps that live in the browser). A typical AngularJS application is composed of HTML, CSS, images, and JavaScript files. The web application communicates with a locally hosted .NET 4.5 Web API responsible for the entire system's backend support. The Kiosk user interface is also supported by a Windows service responsible for handling the exit and shutdown requests from the user interface. Exemplary illustrations for the interface screens are depicted in FIGS. 32 - 56.

### 4.7.2 Remote

The remote user interface may be the same web application used for the Kiosk, with any deviations being strictly within the user interface components (i.e., the screens may not look identical). To achieve this, a separate set of Remote views (a.k.a. screens) is enabled within the web application when the Remote device is detected. From a functionality perspective, the Remote application utilizes the same controlling logic as the Kiosk. Cordova may be used to package the AngularJS web application as a native Android application.

### 4.7.3 Sitemap

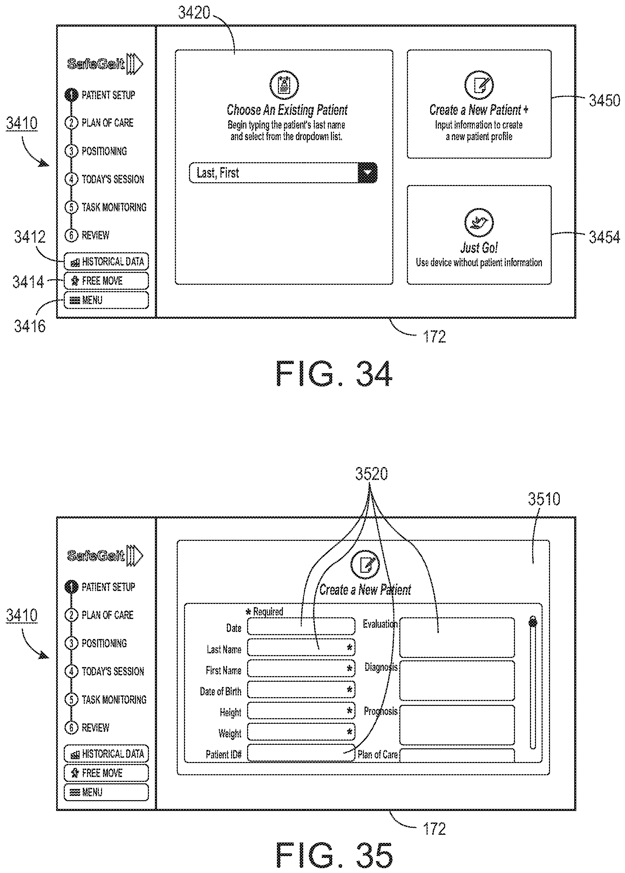

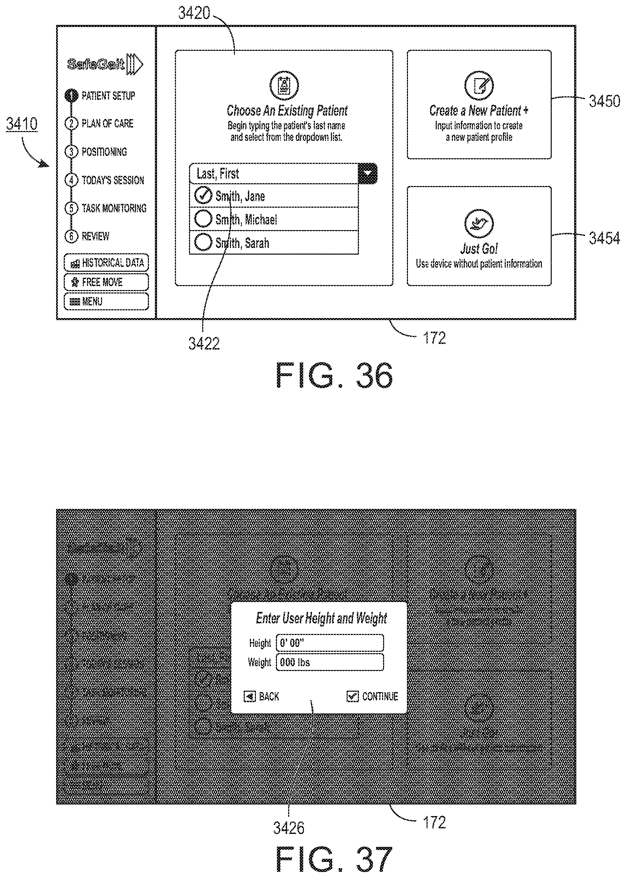

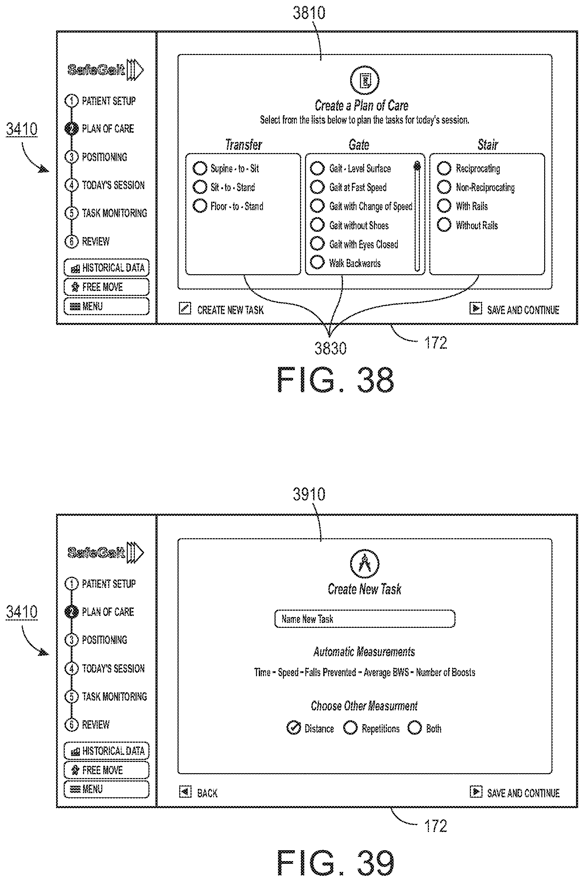

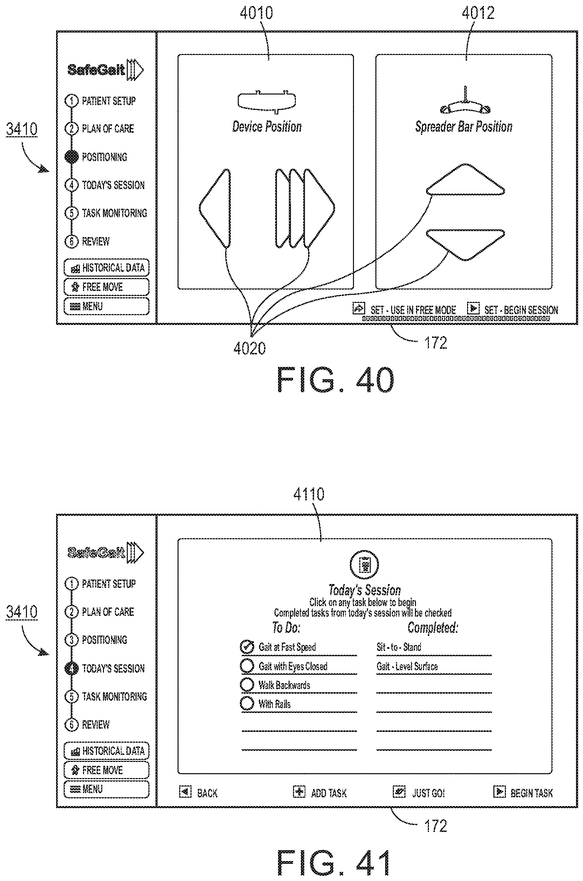

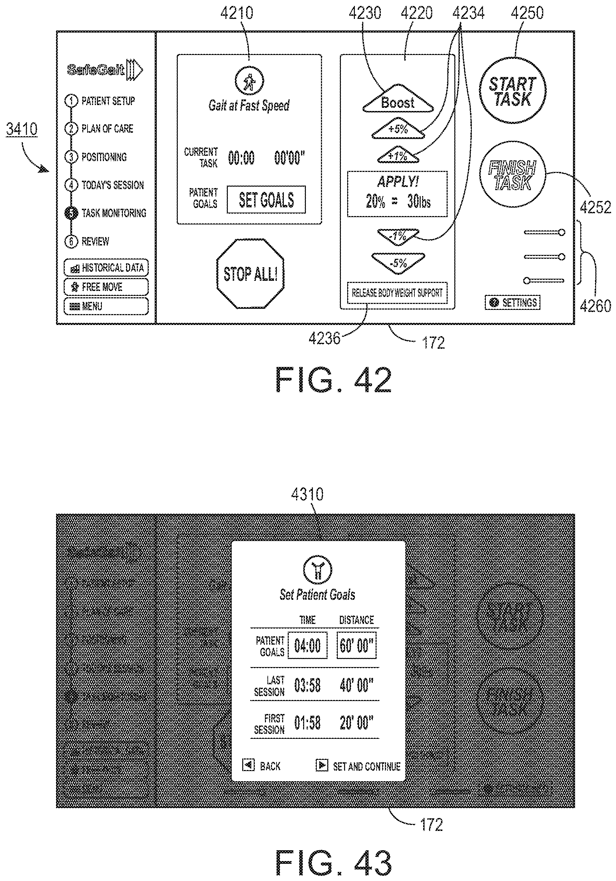

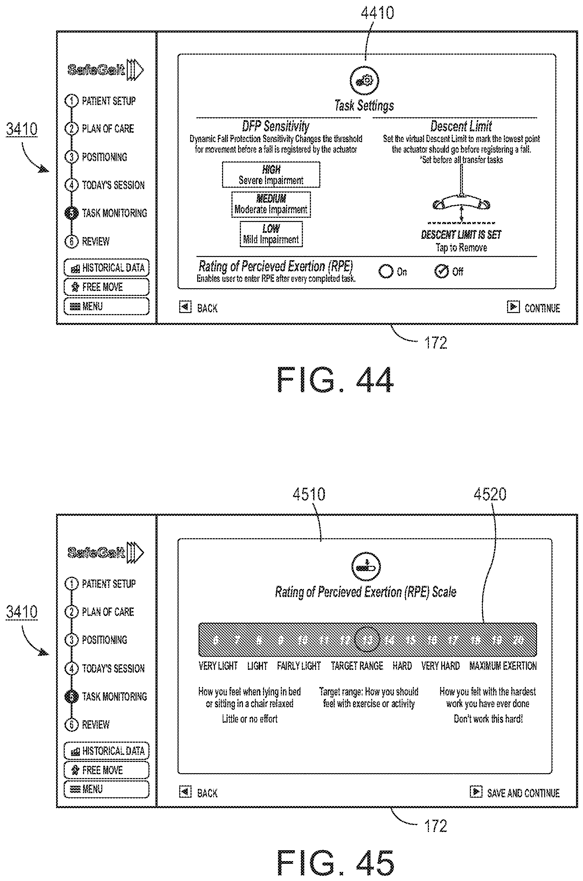

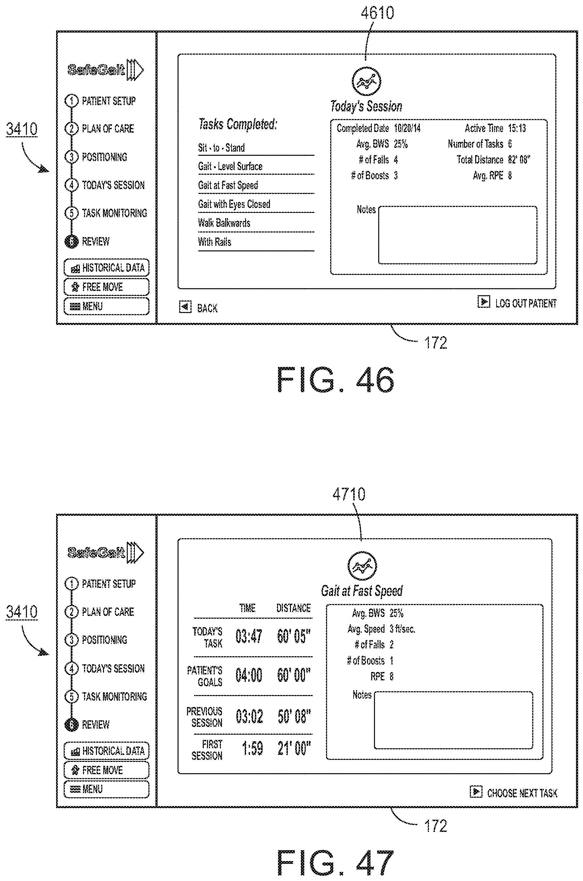

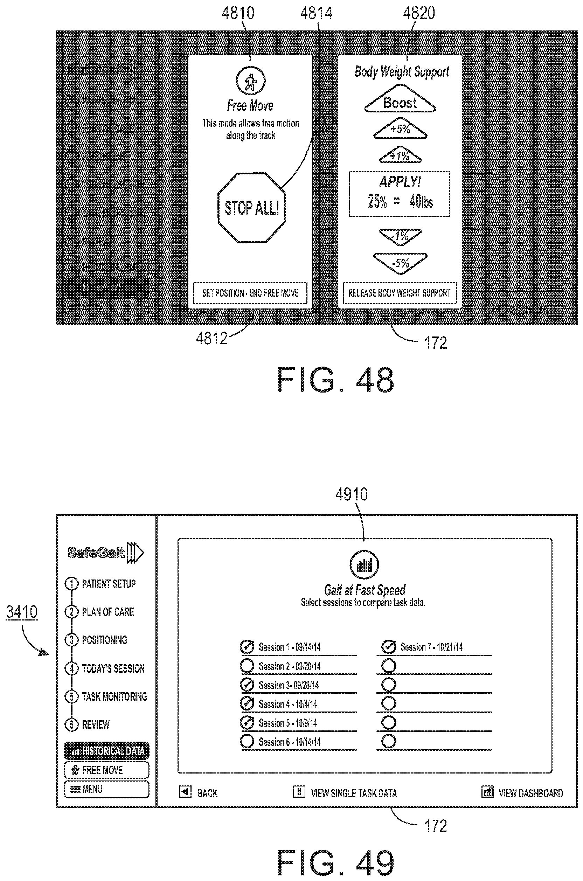

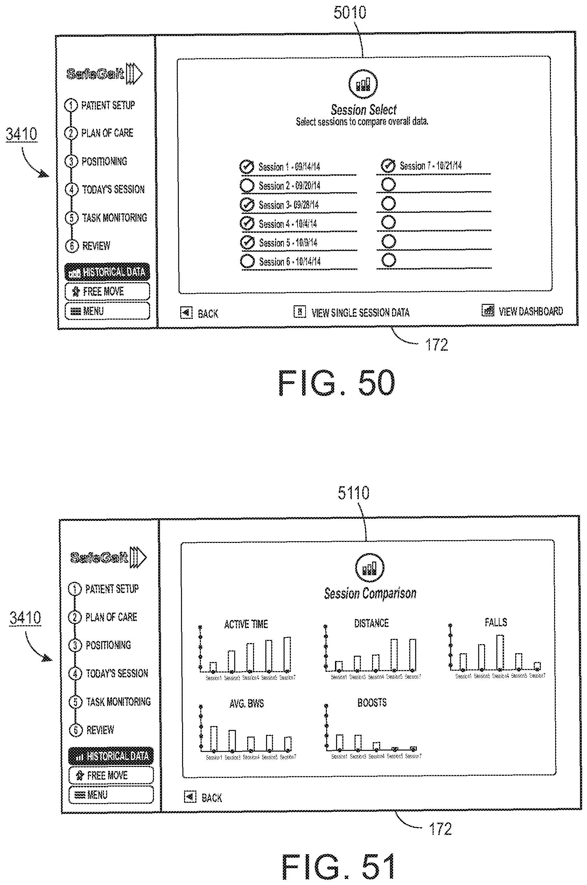

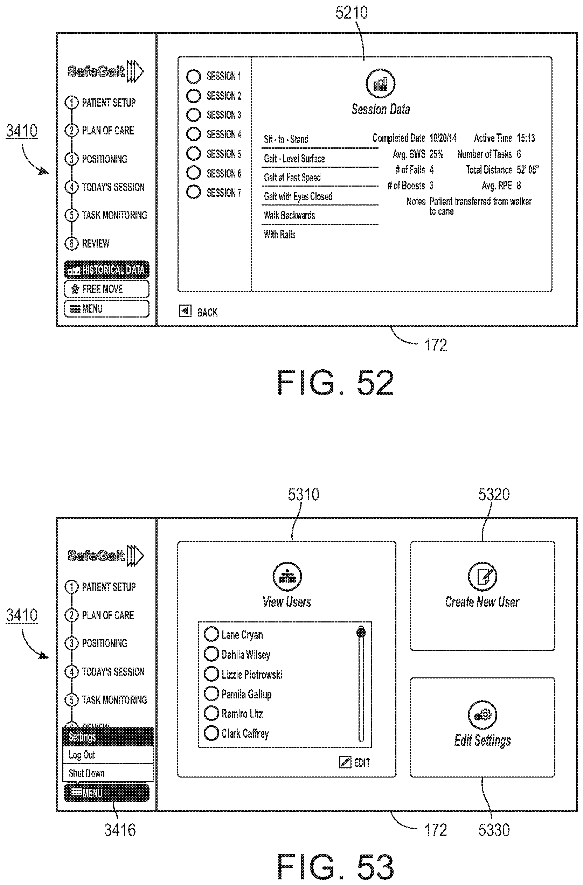

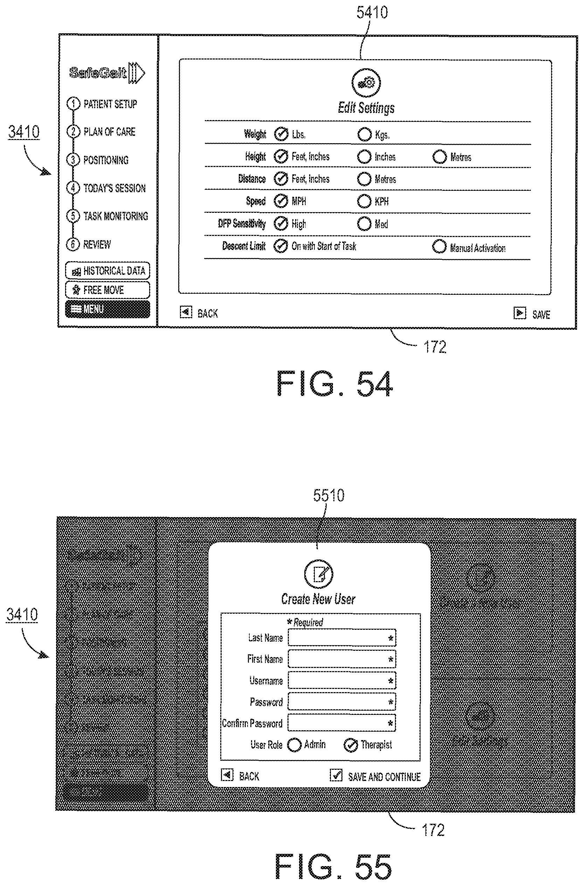

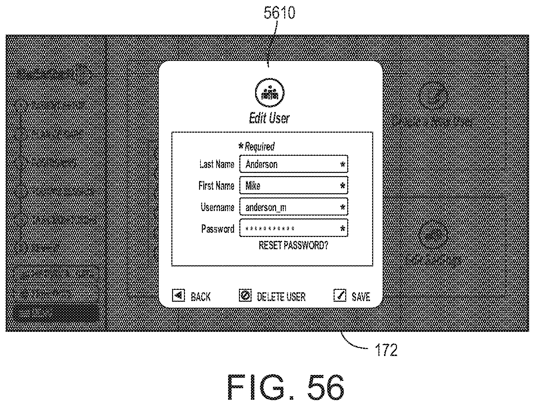

The following sitemap is provided as a snapshot to the user interface design which was used to create the end-user applications for both the Kiosk and Remote. The associated figure numbers for each of the user interface screens/functions are provided.
**4.7.3.1 Startup** - FIG. 32, the user interface **172** shows the status of the system during a start-up process;
**4.7.3.2 Login** - FIG. 33, in the user interface screen 172, username and password fields **3310** are provided for the user to enter data in order to log in to the system;
**4.7.3.3 Patient Setup** - FIG. 34, the user interface screen provides a navigational region **3410** on the left side to show a sequence of operations relative to a patient, as well as buttons to access data (3412), directly operate the actuator (**3414**), or display a menu (**3416**; also see FIG. 53); in the middle of the display is a field **3420** from which to enter/select a patient by name or other form of identification (e.g., patient ID number), and buttons **3450** to select new patient creation, or **3454** to just start a task without patient data;
**4.7.3.3.1 New Patient** - FIG. 35, in response to the selection of Creat New Patient **3450,** the display of a patient information entry screen **3510** and related fields **3520** is facilitated on the interface **172,** and once entered the data can be saved via button **3550** in the lower right corner of the interface;
**4.7.3.3.2 Select Patient (Confirm Height & Weight)** - FIG. 36, in combination with FIG. 37, illustrates a drop-down menu **3422** that facilitates the selection of an existing patient from the database records, and the selection may be followed by the presentation of one or more display windows **3426** that facilitate the entry of a patient's height and weight so that the information may be recorded in the database as well as used to facility setting of one or more of the system variables (e.g., limits, body weight support, boost, etc.);
**4.7.3.3.3 Just Go (Confirm Height & Weight)** - FIG. 37, as discussed above, provides an illustration of the patient height and weight entry window so that information can be input for either an identified patient or in response to the "Just Go" selection **(3454)** of FIG. 34;
**4.7.3.4 Plan of Care** - provides screens so that a therapist can develop a plan of care for the patient using the system;
**4.7.3.4.1 Select Tasks** - FIG. 38 provides the Plan of Care display window **3810,** and includes fields and/or menus **3830** that facilitate the selection of particular components of a task(s) that the patient is to accomplish in the current, or a future, therapy session - where the selected components are also stored in association with the patient's record in the database;
**4.7.3.4.2 Create Custom Task** - FIG. 39 allows a user of the system to create or define a new task via window **3910** to be stored in the system database for use with one or more patients;
**4.7.3.5 Positioning (Actuator** Controller) - FIG. 40 allows the selection of arrow buttons **4020** within either window **4010** or **4012** to facilitate "manual" movement of the movable support or the strap, respectively;
**4.7.3.6 Today's Session** - provides interface screens related to a particular therapy session;
**4.7.3.6.1 Select Task** - FIG. 41 illustrates window **4110** that displays the selections for a particular therapy session and not only allows for the delection of particular tasks (left side), but also illustrates the completed tasks (right side), and the window is closed in response to one of the commands depicted along the bottom of the display in interface **172** (e.g., "Add Task" would add a task to the ToDo list;
**4.7.3.7 Task Monitoring (Actuator Controller)** - FIG. 42 is an exemplary display interface screen showing the information related to control of the actuator on the movable support, and includes display window **4210** showing a currently programmed task as well as the time and/or distance over which the task is performed, and display window **4220** that indicates the level of body weight support being supplied to the patient via the actuator and strap attached to the harness; also included in **4220** is a Boost button **4230** to facilitate a brief increase to the body weight support, buttons **4234** to adjust the level of body weight support, and button **4236** to release (0%) the body weight support feature; buttons **4250** and **4252** provide for respectively starting and stopping the programmed task, and sliding scales **4260** illustrate the setting levels for other parameters of the system;
**4.7.3.7.1 Task Goals** - FIG. 43 illustrates a display window **4310** that allows for the display of past patient performance metrics for the task being performed as well as setting goals for the current session
**4.7.3.7.2 Actuator Settings** - FIG. 44 provides a display window **4410** that permits the adjustment of machine (actuator) parameters such as dynamic fall prevention (DFP) sensitivity (low, medium, high), limits on the height (descent limit) at which the system declares the patient to be falling and triggers a stop to the descent, and means for allowing the system to track a user-entered exertion score (RPE) upon completion of each task (see FIG. 45);
**4.7.3.7.3 RPE** - FIG. 45 is a perceived exertion scale window **4510** and an associated selector (numeric indicator along a range bar **4520** that facilitates entry of a patient's perceived exertion level;
**4.7.3.8 Review** - provides the ability for the therapist or user to review particular sessions or tasks and data associated therewith;
**4.7.3.8.1 Session** - FIG. 46 is an exemplary illustration of a session summary display window **4610** that depicts not only the tasks completed but other information related to the therapy session;
**4.7.3.8.2 Task** - FIG. 47 provides a window **4710** that illustrates particular detail for each of the tasks indicated in the left side of window **4610,** and thereby allows a user to scroll through the tasks and see additional information;
**4.7.3.9 Free Move (Actuator Controller)** - FIG. 48 is an illustrative example of a free movement display interface where the support system operates in a free movement mode **4810** and is available to provide an adjustable level of support for the patient, including button **4812** to set the position of the movable support (e.g., along a track), and Stop button **4814** and window **4820** to control the level of support like window **4220** described above;
**4.7.3.10 Historical Data -** provides screens by which the therapist or user can search or sort stored data within the database;
**4.7.3.10.1 Select Task Filter** - FIG. 49 illustrates a display window **4910** that permits the selection of particular sessions from the database for the comparison of historical performance data;
**4.7.3.10.2 Select Sessions** - FIG. 50 illustrates a display window **5010** that permits the selection of particular sessions from the database for the comparison of historical performance data
**4.7.3.10.3 Chart Dashboard** - FIG. 51 provides an illustrative example of the historical data from sessions selected in FIG. 49 in the nature of bar graphs presented in window **5110;**
**4.7.3.10.4 Session Details** - FIG. 52 provides an illustrative example, in window **5210,** of data retrieved from the database for a selected session
**4.7.3.11 Manage** - FIG. 53 is an exemplary representation of an interface screen **172** that permits the user to view and manage users, including not only selection of users **(5310)** but the creation of new users for the system **(5320)** and the editing of system and user settings **(5330)** as well as the menu associated with button **3416;**
**4.7.3.11.1 Application Settings -** FIG. 54 is a representation of an edit setting window **5410** that would be presented in response to a user selection the edit settings operation in window **5330,** and includes settings such as units and the selection of performance variables (e.g., DFP sensitivity, limits, etc.;
**4.7.3.11.2 Create User** - FIG. 55 is an exemplary representation of a new user creation display window **5510;** and
**4.7.3.11.3 Edit** User - FIG. 56 is an exemplary representation of the edit user display window **5610.**

### 4.8 Installation Packages

Installation packages may be created for the Kiosk software, which is an all-inclusive installer that manages the installation of the following software entities: WebAPI (web services); Database; Kiosk User Interface Application; A means to install the Remote software. Installation requires some configuration, and as stated previously, configuration of the Kiosk (and Remote) at the operating system level is believed to be within the scope of knowledge of one skilled in the art of programmable interface devices.

Having described the programmable user interface in accordance with an embodiment of the system, attention is returned to the general operation of the system. Although described above in relation to FIG. 1 as a rack and pinion type of indexing mechanism, it will also be appreciated that alternative methods and devices may be employed for reliably controlling the horizontal position of the support **130** relative to the track, including the friction drive mentioned and further described with respect to FIGS. 4 - 10.

In one embodiment, an optical receiver/transmitter pair and sensor may be employed to track the position of the support, where a sensor detects an encoded position along the track. As described in more detail, the ability to reliably control the position of the support enables the system to assure its position relative to stations or regions of the track/path (e.g., FIG. 17) are accurately determined. Accurate tracking of the movable support's position permits the potential for use of multiple units on a single track - thereby permitting a plurality of patients to use the same track simultaneously where the units can communicate with one another or with a central position control in order to assure that an appropriate spacing is maintained between adjacent units at all times. In an alternative embodiment, the individual support units themselves may include sensors or other control logic that prevents the units from coming into contact with one another while in operation.

It will be appreciated that although the horizontal position of support **130** is under the control of the horizontal drive, and the support itself otherwise freely slides or rolls along the path defined by the track **120.** The support is connected to roller assembly **128** located on the interior of the track which provides rolling contact with at least the bottom interior of the C-shaped track, and the sides as well. Moreover, the interior of the track may be any conventional track, including a single piece of track or a collection of multiple pieces (e.g., oriented end-to-end). The track may also have electromechanical contacts therein (not shown) that are available to provide electrical power and/or signals to the drives and/or control mechanisms associated with the support. In other words, the roller assembly provides a means for operatively attaching the support to the track, yet minimizing friction using the associated roller assemblies.

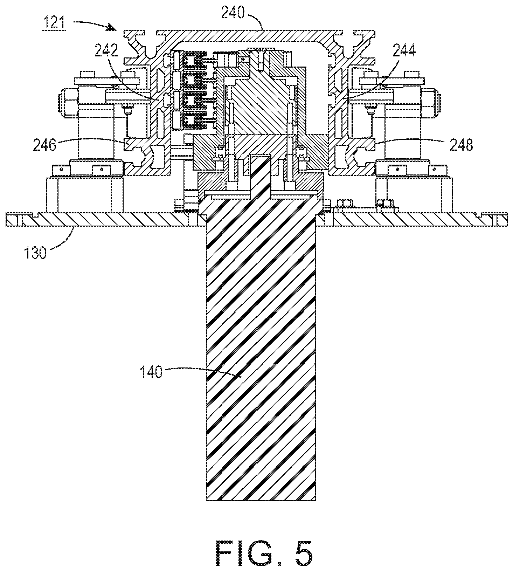

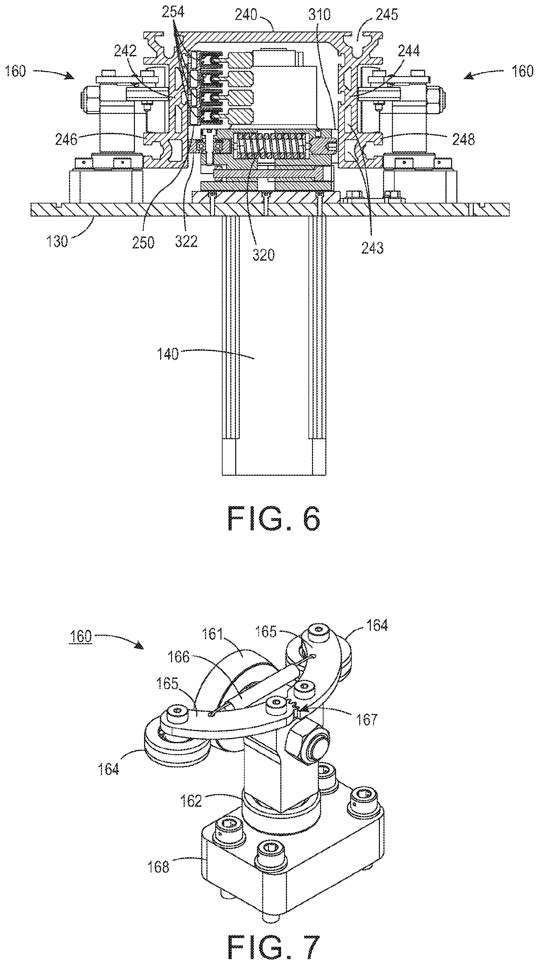

In an alternative configuration such as that depicted in FIGS. 4 - 10, the components of the system are modified to provide a track where support is provided on the exterior of the track and the drive and power interfaces are located on the interior surfaces of the track. As illustrated, for example in FIGS. 4 - 6, the alternative track **121** comprises an assembly of a plurality of extruded members joined end-to-end. The track cross-section is illustrated in FIGS. 5 and 6 which show, respectively, sectional views **5-5** and **6-6** of FIG. 4.

The track includes a generally planar upper web or surface **240,** extending in a longitudinal direction. From the upper web **240,** opposing sides **242** and **244** extend in a downward directed along each side of the upper web. The combination the upper surface and downward-extending sides form the interior portion of the track **121.** Each of said opposing sides further includes a shoulder **246, 248,** respectively, extending in an outward direction therefrom, where the shoulders are oriented perpendicular to the respective side. As further illustrated in the cross-sections, the track includes one or more enclosed channels **243** extending the entire length of each of the downward-extending sides, where the channels reduce the weight and increase the rigidity of the track section. The track sections may further include at least one T-slot **245** suitable for the insertion of a mounting component (e.g., screw or bolt head) therein to facilitate installation and suspension of the track from a ceiling or similar structure. Although not depicted, the track sections are designed to be connected end-to-end using studs or similar splicing members (e.g., a cam-lock splice) that span from the end of one member to the adjoining end of the next track member.

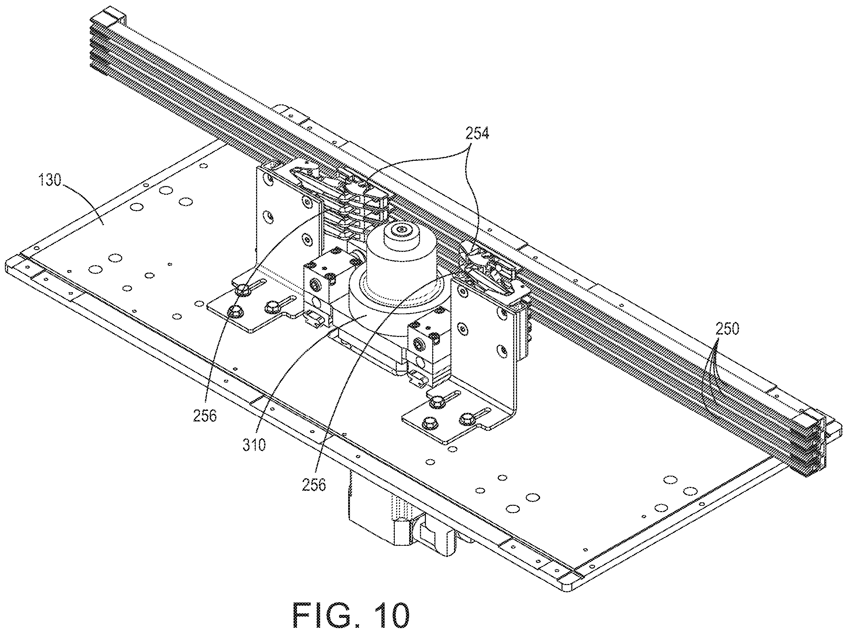

Multiple electric or power rails **250** are spaced along an interior portion of the track along one of the interior sidewalls for each portion of track over which the movable support unit travels. The rails are mounted to the track using insulated standoffs that are attached via internal T-slots provided in the interior of the track sides. Power is transferred from the rails to the control system and motors via one or more shoes **254** that are slidably engaged with the rails, and associated cabling, to ensure power is available. As illustrated in FIG. 10, for example, two shoe assemblies **256** and associated support structures are employed in the system in order to assure continuity of power as the movable support unit **104** travels along the track.

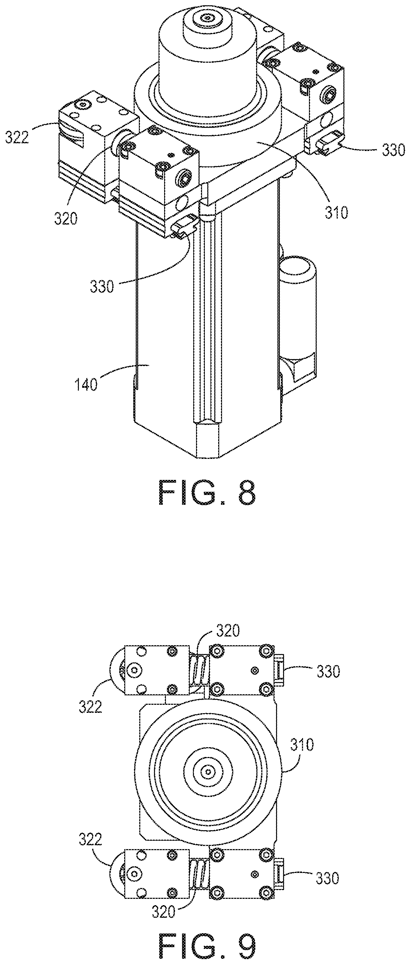

Referring also to FIGS. 6 and 8 - 10, the alternative frictional drive system will be described in further detail. Under the operative control of motor **140,** the frictional drive employs a wheel **310** that is maintained in contact with an inner surface of the track, on the side opposite that which contains the power rails. In other words, the drive wheel **310** is biased away from the power rail side and into contact with the opposite side of the track. The biasing force applied to wheel **310** is supplied via springs **320** and idler wheels **322,** where the idler wheels ride against the interior side of the track and force the drive wheel **310** into frictional contact with the opposite side. The drive assembly (FIG. 8) is allowed to slide or "float" relative to the support **130** as it is operatively coupled to the support **130** via slides **330.** As a result of the disclosed alternative frictional drive mechanism, the first or horizontal drive **140** is slidably connected to the movable support, and the frictional drive mechanism is able to move relative to the support **130,** along a direction that is generally perpendicular to the longitudinal axis of the track.

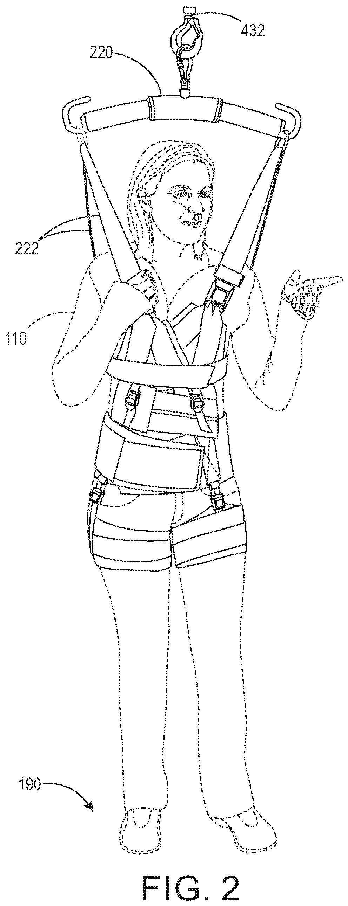

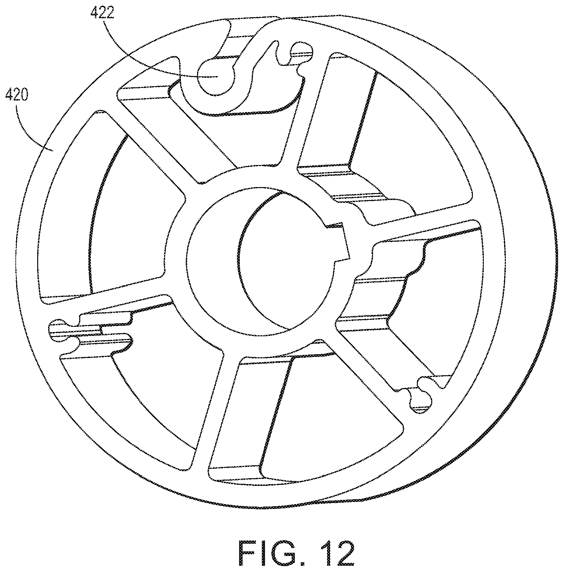

Planar support **130** is intended to be self-centering. That is to say that support **130** is maintained in a horizontal position that is generally centered relative to the track by the combination of at least four suspension assemblies **160** that are depicted in detail in FIG. 7. Each of the assemblies includes a top shoulder wheel **161** and a side shoulder wheel **162,** where the top and side shoulder wheels each maintain contact with respective surfaces of the shoulder (**246** or **248**) extending outward from the track sides. In order to assure that the side shoulder and top shoulder wheels maintain contact and to assure proper tracking of the support, each suspension assembly further includes track idler wheels **164,** along with cammed idler arms **165,** that are pivotally attached to the assembly and operatively connected to one another via a toothed cam **167.** Moreover, arms **165** are biased toward the track side surface that they contact by a spring **166.** In this way the suspension assembly applies an equalizing force to the mounting block **168,** which is in turn affixed to the support plate **130** to cause the plate to self-center during travel and while at rest. Having described the equipment and methodology for driving and controlling the support horizontally, attention is now turned to the balance of the system **100.** Referring also to FIGS. 1-3, 11 and 14, the system further includes an actuator **400** attached to the movable support, where the actuator includes a second drive **410** and associated transmission **412,** such as a worm-gear transmission, connected to and driving a rotatable drum **420.** One advantage of employing a worm gear transmission is the speed reduction of the worm gear is resistant to movement and acts as a braking mechanism should the braking feature of the vertical drive motor **144** fail. The drum **420,** is depicted in perspective view in FIG. 12. The second or belt drive **410** may be an ACOPOS servo drive produced by B&R in Austria (Model #1045) The drum has a strap **430,** having a first end attached in a receptacle **422** and wound about an outer surface of the drum, with a second end of the strap ending in a coupler **432** to connect to a spreader bar **220** and support harness **222** (or similar supportive/ assistive device) attached to support a person **110.** The strap **430,** and as a result the attached spreader bar and/or harness, is raised and lowered under the control of the belt drive **410.** In one embodiment a harness having features such as that disclosed in U.S. Patents 4,981,307 and 5,893,367 (both patents hereby incorporated by reference) may be employed with the disclosed system.

Although an exemplary strap and harness are depicted, it should be appreciated that various alternative harness configurations and support devices may be employed in accordance with the system, and that the intent is not to limit the scope of the disclosed system to the harness depicted. Alternative harness configurations and details may be employed, where several designs of the harness **222** are contemplated without the spreader bar. Harness **222** may include a backplate that is operatively connected, via straps or similar adjustable connections, to a sternum catch pad.

In another contemplated harness design a sternum catch pad has an opening through which the patient places his/her head, such that the pad is placed across the shoulders, over the head and then down the chest or front of the patient where the sternum catch pad is connected to torso pads that are similarly connected to the back plate and extend or wrap around respective sides of the patient's torso. Also, a pair of thigh pads may be provided, each one extending or wrapping around one of the patient's thighs (e.g., below the patient's quadriceps muscles), and each being adjustably connected to the backplate as well as to a connection on the sternum catch pad and/or optional junction pad. In one configuration the design includes a junction pad, a torso pad, sternum catch pad and thigh pads all connect to or through the junction pad. As will be appreciated, the harness **222** may have various configurations. Furthermore, one or more of the backplate and the pads attached thereto may include supporting structures such as metal rods, molded foam padding layers (possibly including impact hardening foam to disperse load forces), straps and associated adjustments and connectors, along with breathable materials such as meshes and the like.

Similarly, the strap **430** may be any elongate member suitable for suspending a person from the system, including rope, cable, etc. having braided, woven, or twisted construction, or possibly even linked or chain-type members. In one embodiment the strap is made from a sublimated polyester, and is intended to provide long life and resistance to stretching. As some therapeutic harnesses are presently adapted for use with strap-type support members, the following disclosure is generally directed to a strap-type member being wound around drum **420.**

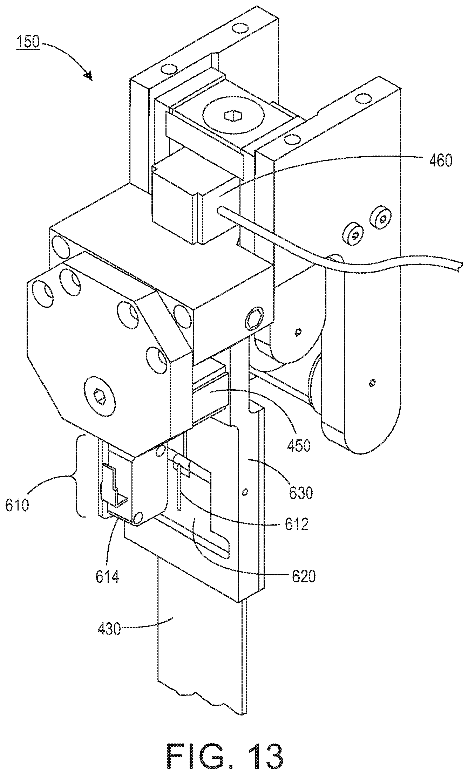

In one embodiment, as depicted in FIGS. 11 and 13 for example, the system includes a first or horizontal load sensor **450** for detecting a horizontal force applied to the support via the strap and a second, or vertical, load sensor **460** for sensing a vertical force applied to the strap. The load cell for the horizontal sensor **450** may be a bi-directional, in-line sensor suitable for axial force measurement.

Sensor **450** may be configured in a manner such that the sensor senses relative position change by a deflection in the downward-extending strap guide. More specifically, as the strap is moved forward or backward in the horizontal direction (H), sensor **450** generates a signal that provides a magnitude of the force applied in the horizontal direction, as well as the direction (e.g., +/-), and outputs the signal to the controller via cable **452.** Thus, in one embodiment the horizontal force detection system detects a horizontal force via the strap using the strap guide operatively attached to and extending from the movable support unit, where the strap guide is operatively connected to a load cell in a manner that results in a change in the load cell output when the strap is pulled in a direction forward from or backward from vertical.

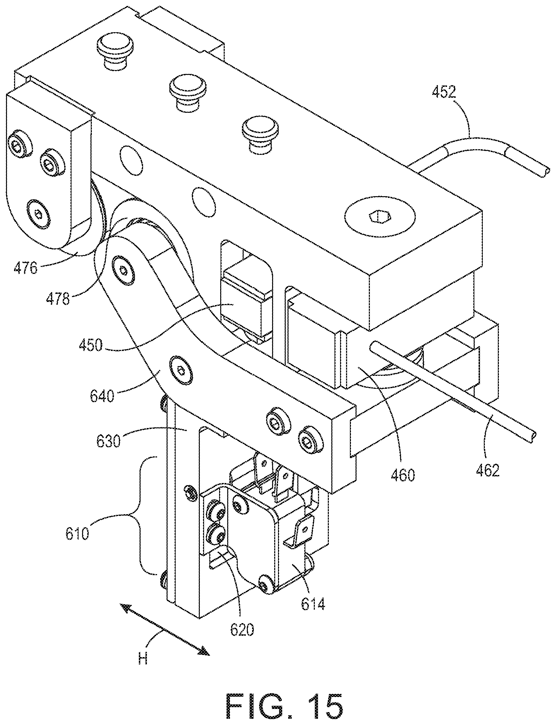

The strap or vertical force sensor **460**, in order to provide increased resolution, may be employed in a compression-only configuration, to sense the force or tension in strap **430**. In the system, the load sensor **460** is used for sensing a downward vertical force (tensile force) applied to the strap, and the sensor assembly includes at least two pulleys or rollers **476** and **478** in a single or double-reeved pulley system **480**. The pulleys are located between the drum and strap guide **630**. As illustrated in FIGS. 14 and 15, for example, the pulley is connected on one end of a pivoting arm **640**: there the arm is pivotally attached near its midsection to a frame member **642** coupled to the movable support plate **130**. The opposite end of pivoting arm **640** is operatively associated with a load cell **460**, so that a downward force applied via strap **430**, results in a similar downward force being applied to pulley or roller **478**. In turn, the downward force is transferred via arm **640** to apply a compression force on the load cell **460**. Thus, load cell **460** is placed only in compression in response to a load suspended on the strap.

In response to signals generated by the load sensors **450** and **460**, a control system, configured to receive signals from the first and second sensors and the user interface **172**, controls the movement of at least the first and second drives to facilitate the support and movement of the person **110**. Moreover, in accordance with one aspect of the disclosed system, the control system dynamically adjusts to provide constant support to the person (e.g., body weight support) via the strap and harness by altering at least the vertical force applied to the strap using the drum and second drive **410**.

With respect to the vertical force, the controller operates, under programmable control to process signals from the vertical load sensor **460** via cable **462**, in combination with prior inputs or pre-set information that sets vertical assistance to be applied to the person via the vertical drive and strap components. For example, the system may have various exercise or therapy modes such as those noted above, whereby the amount of vertical lift or support supplied is adjusted or modified based upon the particular exercise being conducted. For example, walking (gait tasks) over a flat surface the system may control the vertical force to allow the patient to experience about a 90% body weight, whereas on an incline or steps the percentage may be slightly lower, say at about a 70% body weight. To accomplish the control, the system must first determine the patient's body weight - either by sensing it directly in a full support mode or by having the weight (e.g., patient body weight, and optionally spreader bar and harness weight) entered via the user interface. Once determined, the vertical load sensor (load cell) **460** is then employed in a "float" mode to apply an adjusted force of say 10% (100 - 90) body weight to the strap and harness, and thereby reduce force experienced by the patient to approximately 90% of the patient's body weight.

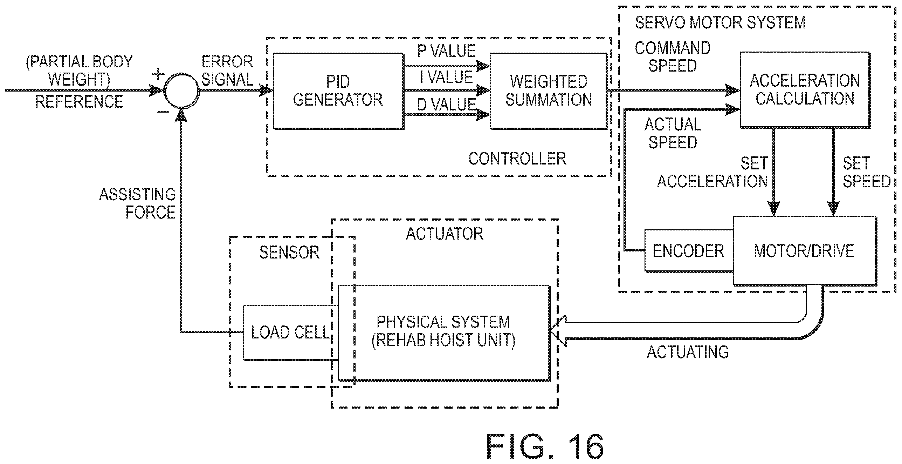

Referring briefly to FIG. 16, depicted therein is a control diagram indicating the relative relationship amongst system components, including the controller, drive servo motor system and the sensor feedback loop The closed-loop control system is applied in both directions (horizontal and vertical) using a PID control technique; proportional (P), integral (I) and derivative (D) gains. Moreover, an acceleration calculation routine is run prior to engaging a motor so that the motion profiles for the system drives are smooth.

In a manner similar to that of the vertical force sensor, horizontal load sensor **450** similarly senses the horizontal component of the load applied to the movable support by the user, via the strap **430.** In this way, when the patient is engaging in an exercise or task that is intended to move along the track or path defined by track **120**, the system **100**, or more particularly the movable support **130** and associated components, may also index or move along the path in order to provide continued vertical support as the patient advances forward or rearward along the path, thereby minimizing the effect of the weight of the unit on the person. Another horizontal load sensing alternative contemplated is the use of a trolley suspension mechanism, with a moment arm associated with the suspended trolley having a load cell attached thereto, to sense changes in the force applied through the moment arm.

In one embodiment, the vertical and horizontal load and position control is accomplished using a programmable controller such as an ACOPOS servo drive, from B&R (e.g., Model #1045). Moreover, the functionality of the controller allows for the control of both the horizontal and vertical positions simultaneously so as to minimize or avoid any delay in the movement and to assure coordination of the control - particularly relative to limits, exercise modes, etc. as further described herein.

Referring also to FIGS. 11 - 15, the belt or strap **430** is wound on a drum **420** in a yo-yo-like fashion, so that the drum contains a plurality of coiled layers of the strap, and is fed through a reeved pulley system **480** to enable the reliable control of the strap and to facilitate sensing forces exerted on the strap. In view of the strap being wound upon itself, the position sensing mechanism associated with the vertical drive operates under the control of an algorithm that automatically adjusts the motion control to account for the change in radius as the strap is rolled or coiled onto and off drum **420.** Also illustrated in FIGS. 13 and 15 is a belt tension sensing system **610**, where a spring-biased arm **612** or similar contactor is in contact with the strap within a window **620** in guide **630**. The arm pivots relative to the guide whenever the strap is slack, and in response to pivoting, the position is sensed by micro-switch **614** and causes a change in the state of the switch. Thus, when the strap is slack (i.e., not taught), the arm pivots under the spring force and the micro-switch is triggered to cause the system to stop further movement in either the vertical or horizontal mode - other than manually controlled movement.

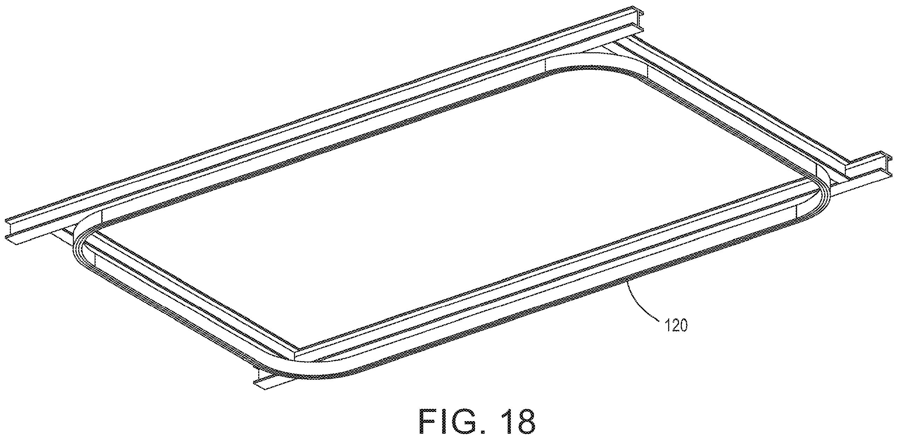

Having described the general operation of the vertical and horizontal load control system, it will be appreciated that this system may be employed to enable multiple exercise modes for the patient such as those described above and represented in FIG. 38. For example, the user interface may be employed to select one or more of such exercise modes to be used. It may also be, as illustrated in FIGS. 17 and 18, that the exercise mode may be controlled via the location of the support relative to the track (e.g., **120**). Referring to FIG. 17, for example, depicted therein is a track **120** that is laid out in a generally rectangular path or course. Along the path are a series of stations or zones **810a** - **810f**, each of which may have one or more exercises to be completed at that station. For example, one station (**810a**) may be designed for walking on a flat surface and may have a set of parallel bars or railings for patient assistance. Another station (**810e**) may have an inclined ramp or stairs that the patient traverses, perhaps at a higher level of assistance (i.e., with a lower percentage of body weight being carried, thus a higher level of vertical support force applied via the strap).

As the movable support moves from one station to another around the loop as illustrated in FIGS. 17 and 18, the type and/or amount of assistance and the nature of the control may be pre-programmed according to the particular zone. It will be appreciated that the locations and characteristics of each zone may themselves be programmable via the user interface and that it is anticipated that loops or paths of varying size and configuration may be customized for the needs of particular patients, therapy centers, etc. And, as noted previously, such information may be stored in the database to facilitate subsequent programming of the system for a particular patient's therapy needs. For example, it may be possible to have a patient's programmatic information stored within a system, and when the patient arrives for therapy, the support system assigned to them is automatically programmed for the same or a slightly modified therapy session from the one that they experienced on their last visit.

As noted above, the use of multiple system units **100** is contemplated in one embodiment. However, it will also be appreciated that the use of multiple systems may require that such systems be able to avoid collisions and/or assure that a buffer space is maintained between adjacent patients. Thus, as illustrated in FIG. 17, the systems, either through a master controller suitable for monitoring the position of all systems, or through intercommunication between the systems themselves, maintain information related to the relative position of adjacent devices such that they maintain a safe separation distance D between the units. Although not illustrated, in the event of a system employing multiple system units, it is further contemplated that one or more units may be "parked" on a spur or other non-use location when not in use in order to allow unimpeded use of the entire therapy circuit by only a single user.

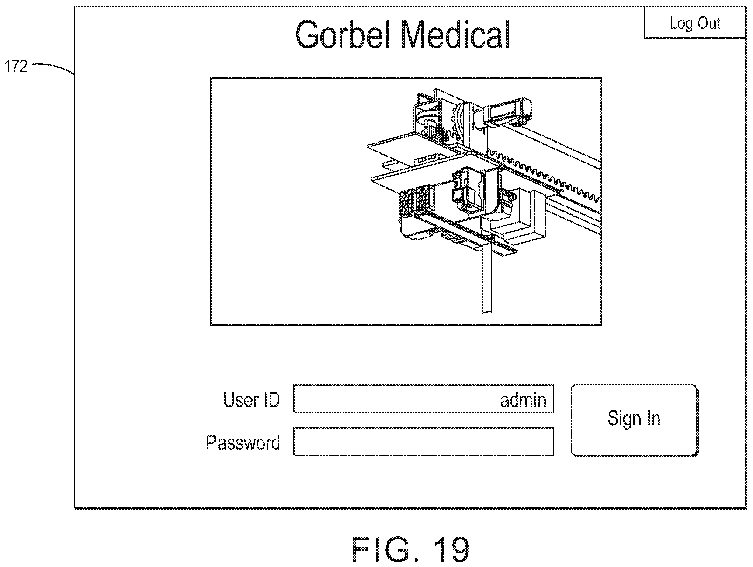

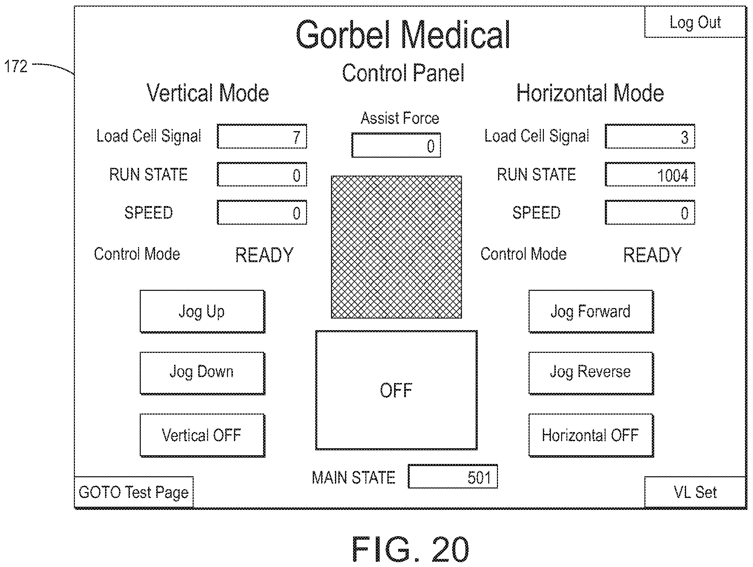

Referring again to FIGS. 19 - 21, depicted therein are exemplary user-interface screens to demonstrate operational features of the disclosed system. The screen depicted on U/I **172** in FIGS. 19 and 33 is a login screen to access the system control pages (interface), several examples of which are found in FIGS. 20 - 21. In FIG. 20, a control panel screen is illustrated for interface **172**. The screen includes information for both the vertical and horizontal controls (modes), including fields indicating the respective load cell signals, run states and speeds. Also indicated is the control mode, in both cases showing READY, to indicate that the system is ready for use of both the vertical and horizontal controls.

In the lower part of the screen of FIG. 20, as well as in FIG. 40, there are shown a series of buttons permitting the manual control of the vertical and horizontal drives, respectively. Each subsystem may be jogged in either direction and the controls for that subsystem may also be disabled. Various system states, including systematic and/or actuator related state numbers, can be displayed for maintenance and/or troubleshooting. Also, the on/off controls for both horizontal and vertical motion are located on this page.

Also contemplated in accordance with the disclosed embodiments are one or more calibration techniques, whereby the various sensors (e.g., vertical load and horizontal force) are calibrated to assure accurate responsiveness to a patient. As noted herein, the load sensors are employed in different configurations and as a result the calibration techniques are also not the same. For example, the vertical force sensor may be employed in a compression-only configuration and thus gives a 1:1 correspondence between the load applied and the output of the load cell. On the other hand, the horizontal load sensor is not a 1:1 relationship to the load. However, the horizontal load sensing is slightly less critical to the operation and support of a patient and therefore a lower resolution/responsiveness may be tolerated for the horizontal load sensor.

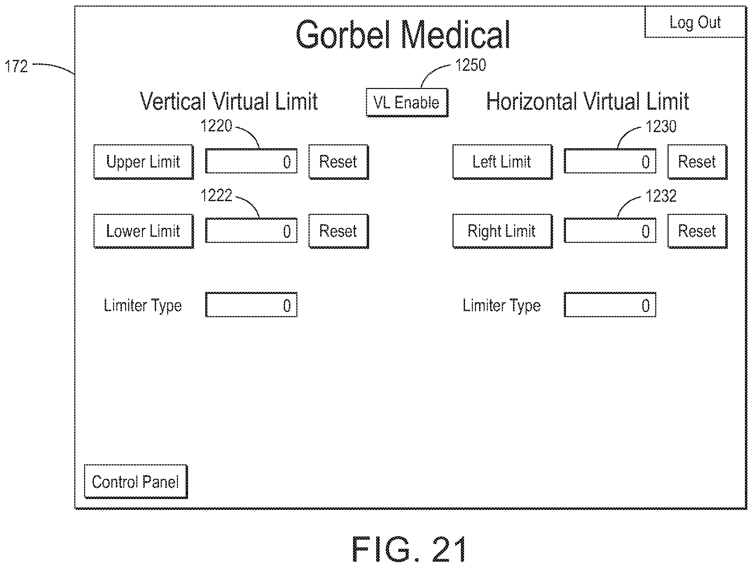

Another feature of the disclosed system is what is referred to as virtual limits. Referring also to FIG. 21, for example, a user interface for the virtual limits is depicted. In one embodiment, there may be several types of limits that are set for a particular system or patient. The limit type may specify a "hard stop" limit, or a soft or transitional limit (where the operation of float mode is adjusted or disabled). For example, in the case of hard stop limits, the limits are set based upon the position - both vertical and/or horizontal. Referring to FIG. 21, the upper and lower limits are entered into fields **1220** and **1222**, respectively. And, use of the reset buttons adjacent to those fields allows the limits to be reset to a pre-determined or default level, or disabled. The left and right limits are similarly entered into fields **1230** and **1232**, respectively, and they may also be reset to a pre-determined or default level or disabled. The depicted interface, as with those depicted in FIGS. 32-56, is responsive to user input via one of many input methods (e.g., touch-screen, mouse, stylus, keyboard, etc.), and the numeric values may be entered into the limit fields via a numeric keypad, scrollable window or other conventional user-interface techniques. Furthermore, such limits may be set by physically manipulating the unit into the position in which the limit is desired to be set, and then recording that location/position. It is further contemplated that the limits themselves may be set for particular zones **810**, and that the values entered may be applicable over the entire system path or only over a portion (zone) thereof. It is also the case that the limits may be enabled or disabled via button **1250** on the screen of interface **172** as depicted in FIG. 21.

The user interface, as further illustrated in FIGS. 35 - 38 for example, is also contemplated to facilitate the collection, storage and display of information related to particular patients, including not only settings for the therapeutic exercises as noted above, but additional information as well. For example, the interface may permit the collection and display of biometric information, user performance metrics, etc. The user interface may be enabled using various technologies in addition to or in place of the standing controller. Examples include wired and wireless devices or computing platforms as well as smartphones, tablets or other personal digital assistive devices, docking stations, etc. Moreover, the computing and/or control resources for the rehab body weight support system may reside in the kiosk controller **170**, in the individual system units themselves, or in other locations that are easily accessed and interconnected through one or more wired or wireless connections.

The SafeGait™ 360° Balance and Mobility Trainer system also contemplates incorporating reporting functionality in the associated interfaces and storage devices in order to provide one or more of the following reports or outputs:
**Patient Record** - Patient information is input by the user that can include demographic information, (Name, DOB, Height, Weight) provider information, (Subscriber ID) and clinical information (Prognosis, Date of Injury, Plan of Care, Progress Notes).
**Task Outcomes** - Provides task specific performance measures, (distance-for a gait task, Repetition- for a transfer task) in addition to non-task specific performance data, (time, # falls prevented, # BOOSTs (see BOOST description below), avg. body weight support (BWS) and speed. Therapists also have the option to capture the patient's rated perceived exertion.
**Session Outcomes** - As illustrated, for example in FIG. 47, the session record or report provides aggregate performance measures for an entire session: Active time, (time patient was engaged in tasks) distance, repetitions, # falls prevented, # BOOSTs, avg. BWS & Speed
**Historical Comparison** - Allows therapists, patients and others to select and graphically compare historical performance by task and session.

Although generally described relative to patient data, also contemplated in the reporting features is the storage and reporting of data relative to the operation of the system(s) (e.g., number of patient records, cumulative usage of the system(s), usage time by therapist, etc.) for purposes of tracking the performance of the system(s) as well.

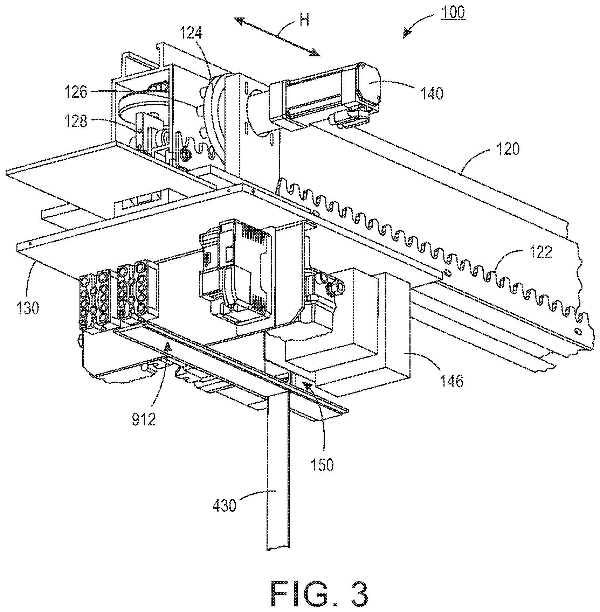

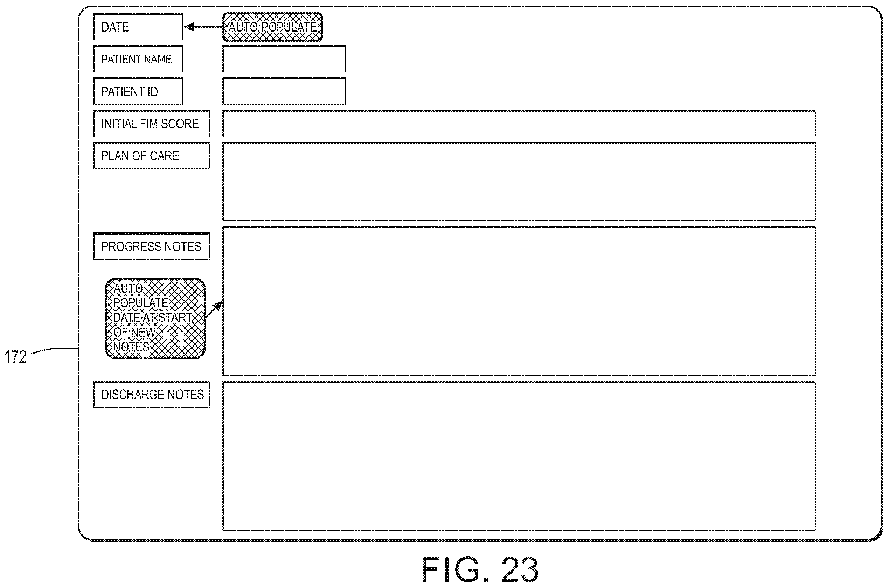

In one embodiment, in addition to a user interface, the system, particularly the movable support unit **104**, may include one or a plurality of indicators such as light-emitting diodes (LEDs) that are under the control of and operated by the control system. The indicators may be provided on any external surface or housing of the support unit, and would be located in a position (e.g., FIG. 3, location **912**) where they would be readily visible to a therapist and/or user of the system in order to provide a visual cue while the therapist is watching the patient using the system. The indicators would display an operational status of the system, and may further signal faults or other information based upon the LED color, mode (e.g., on, off, flashing speed) and combination with the other LEDs. As noted above, the user interface may include handheld as well as any permanently located devices such as touch screens and the like, may also be suitable for displaying information from the control system, and receiving information entered by a therapist to control an operation of the system (see e.g., FIGS. 19 - 21). As further illustrated by FIGS. 22 - 26, the system may include additional computing resources, such as memory or storage devices that enable the storage of data associated not only with system operation, but patient data as well. In one embodiment, the system includes an operation database for storing information relative to the operation of the system. Such a database may also store information relating to use of the system by different patients and their therapists. For example, FIGS. 22 - 23 are illustrative examples of user interface windows that may be used for tracking and entering patient-specific information relating to use of a rehab body weight support system. As shown in FIGS. 22 and 23, various fields are provided to both display and to enter patient information (or have it automatically populated from the database). Certain fields include patient record information for review by the therapist (e.g., date of injury, medical history, prognosis, medications in FIG. 22) while other fields allow the therapist to input information based upon the patient's use of the system (e.g., Initial FIM score, plan or care, progress notes, and discharge notes as illustrated in FIG. 23).

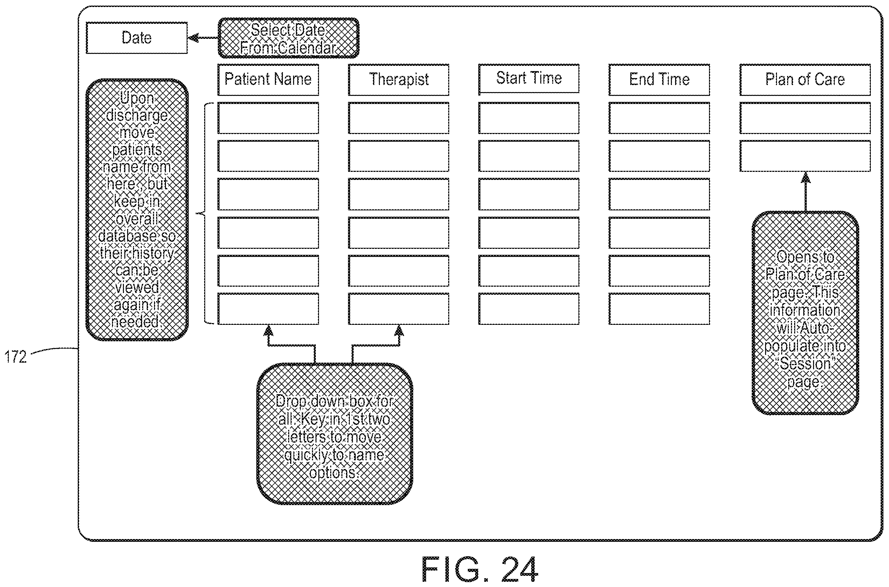

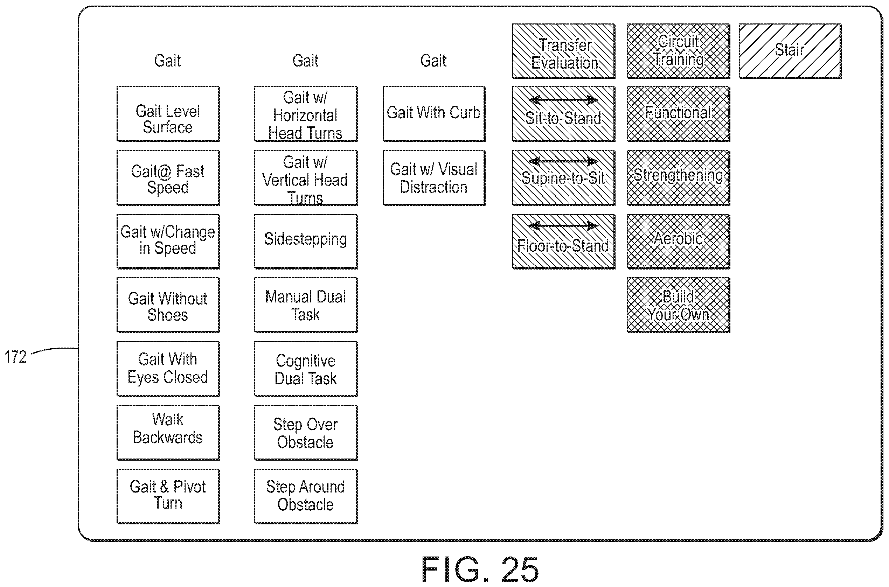

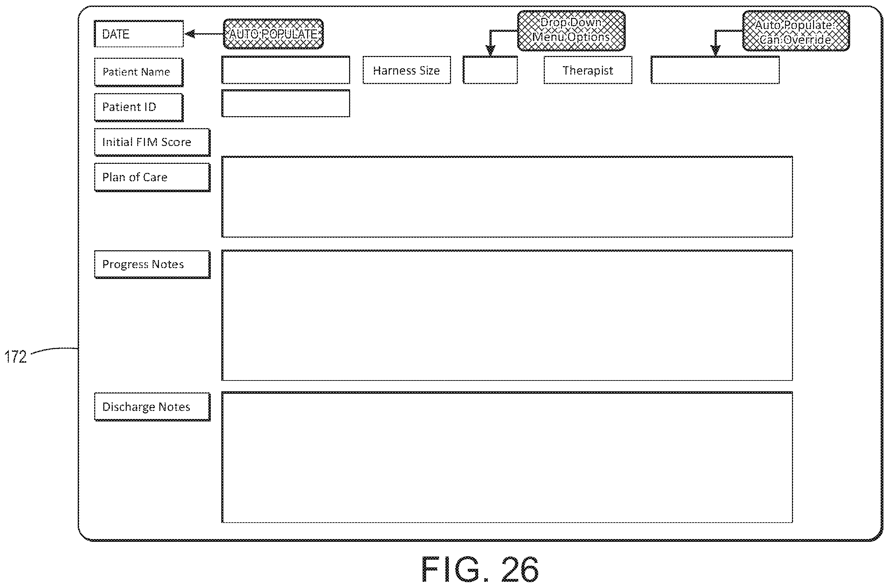

Referring briefly to FIG. 24, there is shown an illustrative example of a user interface **172** depicting a day list window that represents scheduling or usage of the system. As noted, some of the fields depicted on the interface window **172** of FIG. 24 may auto-populate from information contained in the system database, whereas other fields may be drop-down or similar data entry fields that are available to a therapist or other user of the system. Similarly, FIG. 25 provides an illustrative example of a user interface plan of care window on the user interface **172.** In the plan of care window, a therapist may select from one or more pre-programmed activities for the patient. It will be appreciated that the various activities are subject to programmatic control and the input of certain patient-specific information that may be entered or previously stored in the database. Lastly, FIG. 26 provides an illustrative example of a user interface window for review and entry of data for a patient session. Once again, certain fields may be pre-populated with information based upon the patient ID or similar unique identifier. And, the patient session interface also includes fields for the therapist to enter information. It will be understood that the use and display of information is not limited to the particular interface screens depicted. Moreover, the system may also be able to track a patient's performance in order to measure the number of reps, amount of assistance, number of falls prevented, etc. in order to provide such data in the future, or as a performance measurement over time. The dynamic fall prevention aspects of the disclosed embodiments, particularly when the system controller is operated in what is referred to as a float mode, permits the sensing of dynamic fall events, and while preventing actual falls, the system can also log the occurrences for subsequent review and tracking.

in yet another embodiment, the SafeGait™ 360° Balance and Mobility Trainer system further includes or facilitates several additional features. The following features may be implemented on system **100** or a similarly configured system for providing assistance:
**Dynamic Fall Protection** - Dynamic Fall Protection (DFP), as briefly mentioned above, and as represented in FIG. 44, enables the device to sense a fall instead of, or in addition to, setting a pre-defined fall limit (Descent Limit). This makes the response of the SafeGait system more intuitive for a therapist who does not need to pre-determine a height for fall arrest. It also provides fall protection in cases where a Descent Limit cannot be set or may have been set very low; for instance transfer tasks such as supine-to-stand requiring the patient's full vertical range of motion. When a fall is detected with DFP, further vertical movement in the downward direction is inhibited. The patient is allowed movement in only the upward direction and horizontally, albeit at a reduced speed, making it easier to right themselves.
**Dynamic Fall Protection (DFP) Sensitivity Levels** - The DFP Sensitivity Levels are set via an interface screen such as FIG. 44, and allows therapists to adjust fall protection sensitivity to one of a plurality of sensitivities to accommodate patients at varying stages of independence (e.g., High, Medium, Low). At High sensitivity, a fall is detected with very slight movement in the vertical direction. On the other hand, at Low sensitivity a fall is only detected with much more drastic vertical movement (approaching freefall).
   There are at least two options for DFP monitoring, including detection of a change in force or a change in speed for the patient being supported by the system. In the force-based option, the force being applied via the strap **430** is continuously monitored for a change that is indicative of a possible fall or other rapid downward movement of the patient. In the speed-based option the speed of the downward vertical movement of strap **430** is monitored, and in the event of the speed exceeding a threshold the motion is dampened or stopped by the system. As will be appreciated, the thresholds applied for the force or speed, in the respective options, are possible variables that may be modified to adjust the sensitivity of the system for fall detection and prevention. Moreover, there may be other system performance metrics that could be used in fall detection (e.g., rapid change in strap angle), and it is also contemplated that a combination of two or more detection options may also be implemented in order to permit the system to detect other "fall" scenarios.
**Descent Limit** - A secondary fall protection feature, referred to as Descent Limit (see FIG. 44), sets a maximum amount of downward travel of the strap 430 (and patient) and the limit is a variable level based on the patient's position and height. The descent limit can be toggled on or off at will during a task, and set to whatever height within the actuator's range is needed for the task to be performed. When a fall is detected with Descent Limit, further vertical movement of the strap in the downward direction is inhibited. The patient is allowed movement in the upward direction and horizontally, albeit at a reduced speed, once again making it easier to right themselves.
**Boost Mode** - The system may be operated by a patient and/or therapist to provide a boost force via the strap during a particular activity that the patient is performing. Engaging the BOOST mode (e.g., via button on interface screen **172** in FIG. 42) provides an additional portion (e.g., fractional percentage) of body weight support for a period of time (e.g., seconds). For example, the system may provide an additional 20% body weight support (BWS) for 10 seconds. And, the amount of boost as well as the time period may be variables that are adjustable by the therapist or user of the system, and may, as described above, be similarly set, adjusted and/or stored on a patient by patient basis. The concept behind BOOST centers on being able to assist a patient in completing a task as they fatigue, (i.e. a final sit-to-stand repetition). BOOST mode allows the therapist to offer the patient added BWS for a short duration with a single action, (one click) as opposed to adjusting the body weight support settings twice (e.g., first adjusting it upward for more support followed by quickly adjusting it back to the previous level).

Also contemplated in the disclosed embodiments is the automatic population of certain pieces of operational information (fields), as well as operational settings for the system, based upon not only the information stored in the database, but the entry of data by the therapist as well. As a result, a user interface such as the examples set forth would be available to a therapist or other user of the system, and may display information selected in the form of a patient record window, a day list window showing use of the system, a plan of care selection window and/or a session data window.

## Claims

1. A system for supporting a person, comprising:
a movable support unit (130) operatively associated with and moveable along or relative to a support system (120), the movable support unit (130) being movable along a path;
at least a first drive (140) associated with the movable support unit (130), said first drive moving the support (130) along the path;
an actuator (400) attached to the movable support unit (103), said actuator (400) including a second drive (410) for driving a rotatable drum (420), said drum (420) having a first end of a strap (430) attached thereto, said strap wound in an overlapping coil fashion about an outer surface of the drum (420), and a second end of the strap being coupled to a support harness (222) attached to support the person;
a first sensor (450) configured to measure a magnitude and a direction of a horizontal force applied to the movable support unit (130) via the strap (430);
a second sensor (460) configured to measure, as a tensile force, at least a magnitude of a vertical force applied to the strap (430); and
a control system configured to receive signals from the first and second sensors (450, 460), and a user interface, the control system configured to control, in response to the received signals, the movement of at least the first and second drives to facilitate the support during movement of the person, where the control system, in combination with at least one of the first sensor (450) and the second sensor (460), and in combination with a user interface (172), provides dynamic fall protection wherein upon sensing a fall as a change in the output from one of the first sensor (450) and the second sensor (460) the control system dynamically adjusts the vertical force applied to the person via the strap (430), the drum (420) and the second drive (410) where the control system further includes selectable dynamic fall prevention sensitivity levels to alter the amount of change in force sensed by at least one of the first sensor (450) and the second sensor (460) necessary to trigger the dynamic fall prevention and prevent a fall by preventing further movement of at least the second drive to inhibit the person's movement in the downward direction; and
a database for storing data representative of the operation of the system, said database being associated with the control system and where the user interface directs queries to said database, said system providing, in response to a query, at least one report selected from the group consisting of: a patient record, task outcomes, session outcomes, and historical comparisons.

2. The system according to claim 1 further comprising:
a plurality of indicators, operated by the control system, said indicators displaying an operational status of the system; and
said user interface further displaying information from said control system and said database, and also receiving information entered by a therapist to control at least one operation of the system.

3. The system according to claim 1, wherein the database stores information relative to the operation of the system and where the user interface further displays information in a format selected from the group consisting of: a patient record window; a day list window showing use of the system; a plan of care selection window; and a session data window.

4. The system according to claim 1, further comprising a boost mode, wherein when the system operates to support a portion of the body weight of the person, a temporary increase in the amount of body weight support is applied by the system using the strap, the drum and the second drive for a defined period of time .

5. The system according to claim 1, wherein the path is defined by a track, and where the first drive (140) is frictionally coupled to a surface of the track to control the horizontal position of the support (130) along the track.

6. The system according to claim 1, wherein said user interface comprises at least one computing system spaced apart from said movable support, said computing system is in wireless communication with said moveable support, wherein said interface displays stored data for the person, retrieved from said database, to facilitate the selection of at least one therapy task for the person.

7. The system according to claim 6 wherein said interface displays at least one operational setting selected from the group consisting of: user administration, patient administration; system management; actuator interface.

8. The system according to claim 7 wherein said interface displays at least one user administration operation selected from the group consisting of: Login; Logout; Add User; Update User; Reset Password; Change Password; and Disable/Enable User, where said interface displays at least one patient administration operation selected from the group consisting of: Add Patient; Update Patient; Add Session; Update Session; Get Tasks; Add Custom Task; Get Patient Sessions; Get Patient Goals; and Get Session Task Metrics; wherein said interface displays at least one system management operation selected from the group consisting of: Heartbeat Monitor; Actuator Connectivity Monitor; Kiosk/Remote Heartbeat; Grab System Control; Throw System Control; Get/Set Application Settings; and Exit App/Shutdown; wherein said interface displays at least one actuator interface operation selected from the group consisting of: StopAll; reset; move; stop; get status; get version; get serial number; begin task; end task; enable float mode; enable descent limit; set repetition lower limit; set repetition upper limit; set descent limit height; set body weight support; set Dfp sensitivity; apply boost; and clear fall.

9. The system according to claim 1, wherein the control system further includes, as a descent limit, a maximum amount of downward strap travel, and where the control system inhibits the person's movement in the downward direction.

## Patentansprüche

1. System zum Unterstützen einer Person, welches folgendes aufweist:
eine bewegliche Unterstützungseinheit (130), die einem Unterstützungssystem (120) betriebsfähig zugeordnet und an diesem entlang oder relativ zu diesem beweglich ist, wobei die bewegliche Unterstützungseinheit (130) entlang einem Pfad beweglich ist,
zumindest einen ersten Antrieb (140), der der beweglichen Unterstützungseinheit (130) zugeordnet ist, wobei der erste Antrieb die Unterstützung (130) entlang dem Pfad bewegt,
einen Aktuator (400), der an der beweglichen Unterstützungseinheit (103) angebracht ist, wobei der Aktuator (400) einen zweite Antrieb (410) zum Antreiben einer drehbaren Trommel (420) umfasst, wobei an der Trommel (420) ein erstes Ende eines Gurts (430) befestigt ist, und der Gurt nach Art einer überlappend gewickelten Spule um eine äußere Oberfläche der Trommel (420) gewickelt ist, und ein zweites Ende des Gurts mit einem Unterstützungsgeschirr (220, 222) gekoppelt ist, das zum Unterstützen einer Person angebracht ist,
einen ersten Sensor (450), der zum Messen der Größenordnung und der Richtung einer horizontalen Kraft ausgelegt ist, welche auf die bewegliche Unterstützungseinheit (130) über den Gurt (430) ausgeübt wird,
einen zweiten Sensor (460), der zum Messen, als eine Zugkraft, zumindest der Größenordnung einer vertikalen Kraft ausgelegt ist, welche auf den Gurt (430) ausgeübt wird,
ein Steuersystem, das dazu ausgelegt ist, Signale von dem ersten und zweiten Sensor (450, 460) zu empfangen, und eine Benutzerschnittstelle, wobei das Steuersystem dazu ausgelegt ist, als Reaktion auf die empfangenen Signale, die Bewegung von zumindest dem ersten und dem zweiten Antrieb zu steuern, um die Unterstützung, während sich die Person bewegt, zu erleichtern, wobei das Steuersystem, in Kombination mit zumindest einem von erstem Sensor (450) und zweitem Sensor (460) und in Kombination mit der Benutzerschnittstelle (172), einen dynamischen Sturzschutz bereitstellt, wobei auf das Detektieren eines Sturzes als eine Änderung in der Ausgabe von einem von erstem Sensor (450) und zweitem Sensor (460) hin das Steuersystem dynamisch die vertikale Kraft einstellt, die auf die Person über den Gurt (430), die Trommel (420) und den zweiten Antrieb (410) ausgeübt wird, wobei das Steuersystem ferner auswählbare dynamische Sturzpräventionsempfindlichkeitsstufen umfasst, um den Betrag der von zumindest einem von erstem Sensor (450) und zweitem Sensor (460) detektierten Änderung der Kraft zu verändern, der notwendig ist, um die dynamische Sturzprävention auszulösen und einen Sturz durch Verhinderung weiterer Bewegung zumindest des zweiten Antriebs zum Hemmen der Bewegung der Person in Richtung abwärts zu vermeiden, und
eine Datenbank zum Speichern von Daten, die den Betrieb des Systems repräsentieren, wobei die Datenbank dem Steuersystem zugeordnet ist, und wobei die Benutzerschnittstelle Anfragen an die Datenbank richtet, wobei das System, in Reaktion auf eine Anfrage, mindestens einen Bericht bereitstellt, der ausgewählt ist aus der Gruppe bestehend aus einem Patientendatensatz, Aufgaben-Ergebnissen, Übungseinheitsergebnissen und historischen Vergleichen.

2. System gemäß Anspruch 1, ferner aufweisend:
mehrere von dem Steuersystem betriebene Anzeigen, wobei die Anzeigen einen Betriebszustand des Systems anzeigen, und
wobei die Benutzerschnittstelle ferner Information von dem Steuersystem und der Datenbank anzeigt und auch Information empfängt, die von einem Therapeuten eingegeben wird, um zumindest eine Funktion des Systems zu steuern.

3. System gemäß Anspruch 1, wobei die Datenbank Information mit Bezug zum Betrieb des Systems speichert, und wobei die Benutzerschnittstelle ferner Information in einem Format anzeigt, das aus der Gruppe ausgewählt ist, die besteht aus: einem Patientendatensatz-Fenster, einem Tageslistenfenster, das die Benutzung des Systems zeigt, einem Pflegeplanauswahlfenster und einem Übungseinheitsdatenfenster.

4. System gemäß Anspruch 1, ferner aufweisend einen Verstärkungsmodus, wobei, wenn das System zum Halten eines Teils des Körpergewichts der Person betrieben wird, ein vorübergehender Anstieg der Menge an Körpergewichtunterstützung vom System mittels des Gurts, der Trommel und des zweiten Antriebs über eine definierte Zeitdauer ausgeübt wird.

5. System gemäß Anspruch 1, wobei der Pfad durch eine Schiene definiert wird, und wobei der erste Antrieb (140) auf reibende Weise mit einer Oberfläche der Schiene gekoppelt ist, um die horizontale Position der Unterstützung (130) entlang der Schiene zu steuern.

6. System gemäß Anspruch 1, wobei die Benutzerschnittstelle mindestens ein von der beweglichen Unterstützungseinheit beabstandetes Computersystem aufweist, wobei das Computersystem in drahtloser Verbindung mit der beweglichen Unterstützungseinheit steht, wobei die Schnittstelle von der Datenbank abgefragte gespeicherte Daten der Person anzeigt, um die Auswahl mindestens einer Therapie-Aufgabe für die Person zu erleichtern.

7. System gemäß Anspruch 6, wobei die Schnittstelle mindestens eine Betriebseinstellung darstellt, die ausgewählt ist aus der Gruppe bestehend aus: Benutzerverwaltung, Patientenverwaltung, Systemverwaltung, Aktuator-Schnittstelle.

8. System gemäß Anspruch 7, wobei die Schnittstelle mindestens eine Benutzerverwaltungsfunktion anzeigt, die ausgewählt ist aus der Gruppe bestehend aus: Einloggen, Ausloggen, Benutzer Hinzufügen, Benutzer Aktualisieren, Passwort Zurücksetzen, Passwort Ändern, und Benutzer Aktivieren / Deaktivieren, wobei die Schnittstelle mindestens eine Patientenverwaltungsfunktion anzeigt, die ausgewählt ist aus der Gruppe bestehend aus: Patient hinzufügen, Patient Aktualisieren, Übungseinheit hinzufügen, Übungseinheit Aktualisieren, Aufgaben Holen, benutzerdefinierte Aufgabe Hinzufügen, Patienten-Übungseinheiten Holen, Patientenziele Holen, und Messung von Übungseinheitsaufgaben, wobei die Schnittstelle mindestens eine Systemverwaltungsfunktion anzeigt, die ausgewählt ist aus der Gruppe bestehend aus: Herzschlag-Überwachung, Überwachung der Aktuatorverbindungsmöglichkeiten, Kiosk / entfernter Herzschlag, Systemsteuerung Ergreifen, Systemsteuerung Werfen, Holen / Einstellen von Anwendungseinstellungen, und Anwendung Verlassen / Abschalten, wobei die Schnittstelle mindestens eine Aktuator-Schnittstelle-Funktion anzeigt, die ausgewählt ist aus der Gruppe bestehend aus: Alles Anhalten, zurücksetzen, bewegen, halten, Zustand holen, Version holen, Seriennummer holen, Aufgabe beginnen, Aufgabe beenden, Schwebemodus aktivieren, Sinkgrenze aktivieren, Untergrenze für Wiederholungen einstellen, Obergrenze für Wiederholungen einstellen, Höhe der Sinkgrenze einstellen, Unterstützung des Körpergewichts einstellen, Dfp-Empfindlichkeit einstellen, Verstärkung anwenden, und Fall freigeben.

9. System gemäß Anspruch 1, wobei das System ferner, als eine Sinkgrenze, einen maximalen Betrag an Abwärtsweg des Gurts umfasst, und wobei das Steuersystem die Bewegung der Person in Richtung abwärts hemmt.

## Revendications

1. Système pour soutenir une personne, comprenant :
une unité de support mobile (130) associée de manière fonctionnelle à et déplaçable long de ou par rapport à un système de support (120), l'unité de support mobile (130) étant mobile le long d'un trajet ;
au moins un premier dispositif d'entraînement (140) associé à l'unité de support mobile (130), ledit premier dispositif d'entraînement déplaçant le support (130) le long du trajet ;
un actionneur (400) fixé à l'unité de support mobile (130), ledit actionneur (400) comprenant un second dispositif d'entraînement (410) pour entraîner un tambour rotatif (420), ledit tambour (420) ayant une première extrémité d'une sangle (430) fixée à celui-ci, ladite sangle s'enroulant d'une manière hélicoïdale chevauchante autour d'une surface externe du tambour (420), et une seconde extrémité de la sangle étant couplée à un harnais de support (222) fixé pour soutenir la personne ;
un premier capteur (450) configuré pour mesurer une grandeur et une direction d'une force horizontale appliquée à l'unité de support mobile (130) par l'intermédiaire de la sangle (430) ;
un second capteur (460) configuré pour mesurer, en tant que force de traction, au moins une grandeur d'une force verticale appliquée à la sangle (430) ; et
un système de commande configuré pour recevoir des signaux provenant des premier et second capteurs (450, 460), et une interface utilisateur, le système de commande étant configuré pour commander, en réponse aux signaux reçus, le mouvement au moins des premier et second dispositifs d'entraînement pour faciliter le support pendant un mouvement de la personne, le système de commande, en combinaison avec au moins un parmi le premier capteur (450) et le second capteur (460), et en combinaison avec une interface utilisateur (172), fournissant une protection antichute dynamique, dans lequel, lors de la détection d'une chute en tant qu'une modification dans la sortie d'un parmi le premier capteur (450) et le second capteur (460), le système de commande ajuste dynamiquement la force verticale appliquée à la personne par l'intermédiaire de la sangle (430), du tambour (420) et du second dispositif d'entraînement (410), le système de commande comprenant en outre des niveaux de sensibilité de prévention dynamique de chute et pouvant être sélectionnés, pour modifier la quantité de modification dans la force, détectée par au moins un parmi le premier capteur (450) et le second capteur (460), nécessaire pour déclencher la prévention dynamique de chute et empêcher une chute en empêchant un mouvement supplémentaire d'au moins le second dispositif d'entraînement pour bloquer le mouvement de la personne dans la direction vers le bas ; et
une base de données pour stocker des données représentatives du fonctionnement du système, ladite base de données étant associée au système de commande, et l'interface utilisateur orientant des requêtes vers ladite base de données, ledit système fournissant, en réponse à une requête, au moins un rapport choisi parmi le groupe constitué : d'un dossier patient, de résultats d'activité, de résultats de session et de comparaisons d'historiques.

2. Système selon la revendication 1, comprenant en outre :
une pluralité d'indicateurs, actionnés par le système de commande, lesdits indicateurs affichant un statut de fonctionnement du système ; et
ladite interface utilisateur affichant en outre des informations provenant dudit système de commande et de ladite base de données, et recevant également des informations entrées par un thérapeute pour commander au moins un fonctionnement du système.

3. Système selon la revendication 1, dans lequel la base de données stocke des informations relatives au fonctionnement du système et l'interface utilisateur affiche en outre des informations dans un format choisi parmi le groupe constitué : d'une fenêtre de dossier patient, d'une fenêtre de liste de jours montrant une utilisation du système ; d'une fenêtre de sélection de plan de soins ; et d'une fenêtre de données de session.

4. Système selon la revendication 1, comprenant en outre un mode amplifié dans lequel, lorsque le système fonctionne pour soutenir une partie du poids corporel de la personne, une augmentation temporaire dans la quantité de support de poids corporel est appliquée par le système à l'aide de la sangle, du tambour et du second dispositif d'entraînement pendant une période de temps définie.

5. Système selon la revendication 1, dans lequel le trajet est défini par un rail, et le premier dispositif d'entraînement (140) est couplé par frottement à une surface du rail pour commander la position horizontale du support (130) le long du rail.

6. Système selon la revendication 1, dans lequel ladite interface utilisateur comprend au moins un système informatique espacé dudit support mobile, ledit système informatique étant en communication sans fil avec ledit support mobile, ladite interface affichant des données stockées pour la personne, récupérées à partir de ladite base de données, pour faciliter la sélection d'au moins une activité de thérapie pour la personne.

7. Système selon la revendication 6, dans lequel ladite interface affiche au moins un réglage de fonctionnement choisi parmi le groupe constitué : d'une administration d'utilisateur, d'une administration de patient, d'une gestion de système et d'une interface d'actionneur.

8. Système selon la revendication 7, dans lequel ladite interface affiche au moins une opération d'administration d'utilisateur choisie parmi le groupe constitué de : se connecter ; se déconnecter ; ajouter un utilisateur ; mettre à jour un utilisateur ; réinitialiser un mot de passe ; changer un mot de passe ; et activer/désactiver un utilisateur, ladite interface affichant au moins une opération d'administration de patient choisie parmi le groupe constitué de : ajouter un patient ; mettre à jour un patient ; ajouter une session ; mettre à jour une session ; obtenir des activités ; ajouter une activité personnalisée ; obtenir des sessions de patient ; obtenir des objectifs de patient ; et obtenir des mesures d'activité de session ; ladite interface affichant au moins une opération de gestion de système choisie parmi le groupe constitué de : moniteur de rythme cardiaque ; moniteur de connectivité d'actionneur ; rythme cardiaque à distance/kiosque ; récupérer la commande du système ; renvoyer la commande du système ; obtenir/définir des réglages d'application ; et arrêter/sortir de l'application ; ladite interface affichant au moins une opération d'interface d'actionneur choisie parmi le groupe constitué de : tout arrêter ; réinitialiser ; déplacer ; arrêter ; obtenir un statut ; obtenir une version ; obtenir un numéro de série ; commencer une activité ; terminer une activité ; activer un mode flottement ; activer une limite de descente ; définir une limite inférieure de répétition ; définir une limite supérieure de répétition ; définir une hauteur de limite de descente ; définir un support de poids corporel ; définir une sensibilité Dfp ; appliquer une amplification ; et effacer une chute.

9. Système selon la revendication 1, dans lequel le système de commande comprend en outre, en tant que limite de descente, une quantité maximale de déplacement de sangle vers le bas, et le système de commande empêchant un mouvement de la personne dans la direction vers le bas.
